# EUROPEAN PATENT APPLICATION

(11) **EP 4 591 708 A2**
(43) Date of publication of application: **30.07.2025**
(21) Application number: 25169991.4
(22) Date of filing: 08.07.2021
(51) Int. Cl.: A01K 67/0276

(54) **GENE THERAPY FOR STXBP1 ENCEPHALOPATHY**

(30) Priority: 08.07.2020 US 202063049226 P
(62) Divisional of application: 21837304.1
(71) Applicant: Baylor College of Medicine, Houston, TX 77030 (US)
(72) Inventor: XUE, Mingshan, Houston, 77030 (US); CHEN, Wu, Houston, 77030 (US)
(74) Representative: J A Kemp LLP

(57) **Abstract**

The disclosure concerns compositions and methods for treatment of encephalopathies, including encephalopathies caused by, or associated with, and STXBP1 haploinsufficiency or mutation. Compositions and methods provided herein encompass AAV particles that induce expression of STXBP1, including inducing the expression of STXBP1 in central and/or peripheral nervous system cells.

## Description

### CROSS-REFERENCE TO RELATED APPLICATIONS

This application claims priority to U.S. Provisional Patent Application No. 63/049,226 filed July 8, 2020, incorporated herein by reference in its entirety.

### STATEMENT REGARDING FEDERALLY SPONSORED RESEARCH OR DEVELOPMENT

This invention was made with government support under R01NS 100893 awarded by National Institutes of Health. The government has certain rights in the invention.

### SEQUENCE LISTING

The instant application contains a Sequence Listing which has been submitted in ASCII format and is hereby incorporated by reference in its entirety. Said ASCII copy, created on June 26, 2021, is named BAYM_P0313WO_Sequence_Listing.txt and is 20,740 bytes in size.

### TECHNICAL FIELD

Embodiments of the disclosure concern at least the fields of cellular biology, molecular biology, physiology, and medicine.

### BACKGROUND

STXBP1 haploinsufficiency can cause STXBP1 encephalopathy, which is one of the top five causes of pediatric epilepsies, including Ohtahara syndrome, West syndrome, Lennox-Gastaut syndrome, and Dravet syndrome. STXBP1 is one of the most frequently mutated genes in sporadic intellectual disabilities. STXBP1 mutations are often truncating mutations. STXBP1 mutations are typically heterozygous and *de novo.*

Individuals with STXBP1 mutations have intellectual disabilities and the vast majority have epilepsy. STXBP1 encephalopathy leads to motor deficits, including dystonia, ataxia, hypotonia, and tremor. Individuals often have psychiatric defects, including hyperactivity, anxiety, stereotypies, aggression, and autistic features.

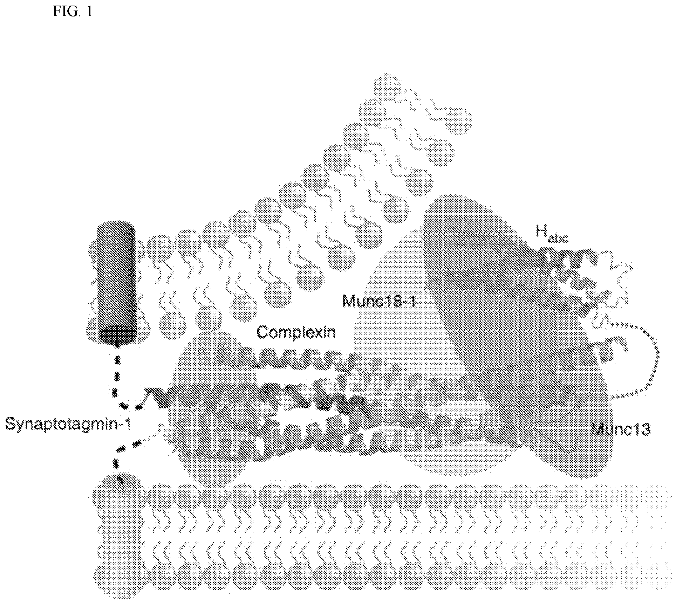

The STXBP1 gene encodes the syntaxin-binding protein 1 (also known as MUNC18-1). It is a core component of neurotransmitter release machinery in all neurons. (FIG. 1) Absence of STXBP1 in model organisms (including worms, fruit flies, and mice) abolishes neurotransmitter release and causes embryonic lethality. Therapies that restore STXBP1 in neurons are needed in the art.

### BRIEF SUMMARY

The present disclosure is directed to compositions and methods for treating encephalopathies of any kind in an individual. In some cases, the encephalopathy may be an epileptic encephalopathy, such as an early infantile epileptic encephalopathy. In some embodiments, the individual has Ohtahara syndrome, West syndrome, Lennox-Gastaut syndrome, Dravet syndrome, or Rett syndrome with mutated *STXBP1.* In some embodiments, the encephalopathy is STXBP1 encephalopathy or early infantile epileptic encephalopathy type 4. The encephalopathy may be caused by, or associated with, a haploinsufficiency in a gene, including STXBP1. The encephalopathy may be caused by, or associated with, one or more mutations in a gene, including STXBP1.

Certain embodiments of the disclosure concern viral particles, including those useful for treating any encephalopathy. In some embodiments, the viral particle is an adeno-associated virus (AAV). The viral particle may transduce cells of the central and/or peripheral nervous system, including neurons, in specific cases. In particular embodiments, the viral particle may transduce any neuron, including for example, glutamatergic neurons, GABAergic neurons, glycinergic neurons, cholinergic neurons, dopaminergic neurons, serotonergic neurons, adrenergic neurons, and/or noradrenergic neurons. In some embodiments, the viral particle is an AAV-PHP.eB particle, AAV-PHP.B particle, AAV-PHP.S particle, AAV-PHP.B4 particle, AAV-PHP.B5 particle, AAV-PHP.N particle, AAV-CAP-B1 particle, AAV-CAP-B10 particle, AAV-CAP-B22 particle, AAV type 1 particle, AAV type 5 particle, AAV type 8 particle, or AAV type 9 particle. In some embodiments, the viral particle is an AAV-PHP.eB particle. In some embodiments, the viral particle is derived from cells transfected with an AAV-PHP.eB construct, AAV-PHP.B construct, AAV-PHP.S construct, AAV-PHP.B4 construct, AAV-PHP.B5 construct, AAV-PHP.N construct, AAV-CAP-B1 construct, AAV-CAP-B10 construct, AAV-CAP-B22 construct, AAV type 1 construct, AAV type 5 construct, AAV type 8 construct, or AAV type 9 construct.

The viral particle may comprise one or more nucleic acids. The nucleic acids may encode for one or more genes that are mutated or haploinsufficient in individuals with an encephalopathy. In some embodiments, the nucleic acid encodes for an isoform of STXBP1. The isoform may be isoform a of STXBP1 or isoform b of STXBP1. In some embodiments, the nucleic acid encodes isoform a of STXBP1. In some embodiments, the nucleic acid encodes isoform b of STXBP1. In some embodiments, the nucleic acid encodes isoform c of STXBP1. In some embodiments, the nucleic acid encodes isoform d of STXBP1.

In some embodiments, the nucleic acid encodes an STXBP1 gene product comprising an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity with SEQ ID NO:1, or any range or value derivable therein. In some embodiments, the nucleic acid encodes an STXBP1 gene product comprising SEQ ID NO:1. In some embodiments, the nucleic acid encodes an STXBP1 gene product comprising an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity with SEQ ID NO:2, or any range or value derivable therein. In some embodiments, the nucleic acid encodes an STXBP1 gene product comprising SEQ ID NO:2. In some embodiments, the nucleic acid encodes an STXBP1 gene product comprising an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity with SEQ ID NO:3, or any range or value derivable therein. In some embodiments, the nucleic acid encodes an STXBP1 gene product comprising SEQ ID NO:3. In some embodiments, the nucleic acid encodes an STXBP1 gene product comprising an amino acid sequence having at least 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity with SEQ ID NO:4, or any range or value derivable therein. In some embodiments, the nucleic acid encodes an STXBP1 gene product comprising SEQ ID NO:4.

In some embodiments, the nucleic acid encoding a gene encodes exons of the gene, including only encodes exons of the gene in some cases. The nucleic acid may lack introns. The nucleic acid may comprise at least one regulatory sequence. In some embodiments, the regulatory sequence, including a promoter, induces constitutive expression, including constitutive expression in neurons. In some embodiments, the viral particle comprises nucleic acids encoding for at least one regulatory sequence and at least one transgene. The regulatory sequence may be capable of inducing constitutive expression of the transgene(s) in any cell

(including neurons) when the cell is transduced by the viral particle. In some embodiments, the viral particle comprises nucleic acids encoding one or more transgenes and comprises one or more regulatory sequences that induce expression of the transgene(s), including specifically in neurons, in some cases. The neurons may be glutamatergic neurons, GABAergic neurons, glycinergic neurons, cholinergic neurons, dopaminergic neurons, serotonergic neurons, adrenergic neurons, and/or noradrenergic neurons. The viral particle may comprise one or more surface markers that cause the specific transduction of the nucleic acid into neurons, including glutamatergic neurons, GABAergic neurons, glycinergic neurons, cholinergic neurons, dopaminergic neurons, serotonergic neurons, adrenergic neurons, and/or noradrenergic neurons. The viral particle may comprise one or more surface markers or other proteins that specifically induce trafficking of the viral particle to the central and/or peripheral nervous system, in some cases.

In certain embodiments, a composition, such as any viral particle encompassed herein, is administered to an individual with an encephalopathy, including STXBP1 encephalopathy. The administration may be by any suitable route or delivery regimen.

The foregoing has outlined rather broadly the features and technical advantages of the present disclosure in order that the detailed description that follows may be better understood. Additional features and advantages will be described hereinafter which form the subject of the claims herein. It should be appreciated by those skilled in the art that the conception and specific embodiments disclosed may be readily utilized as a basis for modifying or designing other structures for carrying out the same purposes of the present designs. It should also be realized by those skilled in the art that such equivalent constructions do not depart from the spirit and scope as set forth in the appended claims. The novel features which are believed to be characteristic of the designs disclosed herein, both as to the organization and method of operation, together with further objects and advantages will be better understood from the following description when considered in connection with the accompanying figures. It is to be expressly understood, however, that each of the figures is provided for the purpose of illustration and description only and is not intended as a definition of the limits of the present disclosure.

### BRIEF DESCRIPTION OF THE DRAWINGS

For a more complete understanding of the present disclosure, reference is now made to the following descriptions taken in conjunction with the accompanying drawing, in which:
FIG. 1 shows STXBP1 (Munc18-1) is part of the neurotransmitter release machinery that includes SNARE proteins (one red, one yellow, and two green helices), Munc13, Complexin, and Synaptotagmin-1;
FIG. 2 illustrates examples of *Stxbpl* constitutive and inducible knockout alleles for mouse models;
FIG. 3 shows STXBP1 protein expression in mice with wild-type, heterozygous, and homozygous tm1d and tmla genotypes. Top panel shows representative Western blots of STXBP1 in wild-type mice and heterozygous and homozygous tmla and tm1d mice. Bottom panel shows STXBP1 protein quantification in wild-type mice and heterozygous and homozygous tmla and tm1d mice. ** p < 0.01, *** p < 0.001, **** p < 0.0001;
FIG. 4 shows a summary of certain phenotypes that are displayed in heterozygous tm1d and tmla mice;
FIG. 5 shows a summary of certain phenotypes that are displayed in heterozygous tm1d and tmla mice;
FIG. 6 shows body weights and hindlimb clasping in wild-type and heterozygous tm1d and tmla mice. *** p < 0.001, **** p < 0.0001;
FIG. 7 shows cognitive function based on a novel object recognition test in wild-type (black and blue symbols) and heterozygous tm1d (red symbols) and tmla mice (orange symbols). Filled circles represent male mice and open circles represent female mice. **** p < 0.0001;
FIG. 8 shows contextual and cued memory in wild-type (black and blue symbols) and heterozygous tm1d (red symbols) and tmla (orange symbols) mice. Filled circles represent male mice and open circles represent female mice. **** p < 0.0001;
FIG. 9 shows motor function based on a foot slip test and a vertical pole test in wild-type (black and blue symbols) and heterozygous tm1d (red symbols) and tmla (orange symbols) mice. Filled circles represent male mice and open circles represent female mice. *** p < 0.001, **** p < 0.0001;
FIG. 10 shows innate psychiatric function based on a nesting test and a marble bury test in wild-type (black and blue symbols) and heterozygous tm1d (red symbols) and tmla (orange symbols) mice. Filled circles represent male mice and open circles represent female mice. **** p < 0.0001;
FIG. 11 shows frequent spike-wave discharges (SWDs, indicated by blue arrows) in EEG recordings from heterozygous tm1d mice;
FIG. 12 shows a quantitative analysis of SWDs. Black symbols are wild-type mice, red symbols are heterozygous tm1d mice. Filled circles represent male mice and open circles represent female mice. **** p < 0.0001;
FIG. 13 shows myoclonic jerks in heterozygous tm1d mice;
FIG. 14 shows myoclonic jumps in heterozygous tm1d mice;
FIG. 15 shows a quantitative analysis of myoclonic seizures. Black symbols are wild-type mice, red symbols are heterozygous tm1d mice. Filled circles represent male mice and open circles represent female mice. ** p < 0.01, *** p < 0.001, **** p < 0.0001;
FIG. 16A shows confocal microscopy images of immunohistochemistry staining of the indicated neuronal markers;
FIG. 16B shows STXBP1 protein expression in adult heterozygous tmla mice with and without PHP.eB viral-transduction of Flpo;
FIG. 17 shows the effect of restoring STXBP1 on hindlimb clasping in adult heterozygous tmla (Mut) mice;
FIG. 18 shows the effect of restoring STXBP1 on cognitive ability based on a novel object recognition test, contextual, and cued memory tests in adult heterozygous tmla (Mut) mice. Filled circles represent male mice and open circles represent female mice. ** p < 0.01, *** p < 0.001, **** p < 0.0001;
FIG. 19 shows the effect of restoring STXBP1 on SWDs in adult heterozygous tmla (Mut) mice. **** p < 0.0001;
FIG. 20 shows STXBP1 protein expression in adult heterozygous tm1d mice with and without PHP.eB viral-transduction of Stxbp1;
FIG. 21 shows the effect of restoring STXBP1 on hindlimb clasping in adult heterozygous tm1d (Mut) mice;
FIG. 22 shows the effect of restoring STXBP1 on cognitive ability based on a novel object recognition test, contextual, and cued memory tests in adult heterozygous tm1d (Mut) mice. Filled circles represent male mice and open circles represent female mice. ** p < 0.01, **** p < 0.0001;
FIG. 23 shows the effect of restoring STXBP1 on motor function based on a foot slip test and a vertical pole test in adult heterozygous tm1d (Mut) mice. Filled circles represent male mice and open circles represent female mice. * p < 0.05, ** p < 0.01, **** p < 0.0001;
FIG. 24 shows the effect of restoring STXBP1 on innate psychiatric function based on a nesting test and a marble bury test in adult heterozygous tm1d (Mut) mice. Filled circles represent male mice and open circles represent female mice. * p < 0.05, ** p < 0.01, *** p < 0.001, **** p < 0.0001;
FIG. 25 shows the effect of restoring STXBP1 on SWDs in adult heterozygous tm1d (Mut) mice. ** p < 0.01;
FIG. 26 shows a summary of certain phenotypes that are displayed in heterozygous tm1d mice or mice with heterozygous knockout of Stxbp1 in either glutamatergic (Vglut2-cHet) or GABAergic (Vgat-cHet) neurons;
FIG. 27 shows myoclonic seizures (jerks and jumps) in wild-type (WT) mice, heterozygous Stxbp1-flox mice (Stxbplf/+), mice expressing Cre in glutamatergic (Vglut2Cre/+) or GABAergic (VgatCre/+) neurons, or mice with heterozygous knockout of Stxbp1 in either glutamatergic (Stxbplf/+;Vglut2Cre/+) or GABAergic (Stxbplf/+;Vgat Cre/+) neurons. * p < 0.05, *** p < 0.001, **** p < 0.0001;
FIG. 28 shows SWDs in WT mice, heterozygous Stxbp1-flox mice (Stxbplf/+), mice expressing Cre in glutamatergic (Vglut2Cre/+) or GABAergic (VgatCre/+) neurons, or mice with heterozygous knockout of Stxbp1 in either glutamatergic (Stxbplf/+;Vglut2Cre/+) or GABAergic (Stxbplf/+;Vgat Cre/+) neurons. * p < 0.05, ** p < 0.01, *** p < 0.001, **** p < 0.0001.

### DETAILED DESCRIPTION

### I. Definitions

In keeping with long-standing patent law convention, the words "a" and "an" when used in the present specification in concert with the word comprising, including the claims, denote "one or more." Some embodiments of the disclosure may consist of or consist essentially of one or more elements, method steps, and/or methods of the disclosure. It is contemplated that any method or composition described herein can be implemented with respect to any other method or composition described herein and that different embodiments may be combined.

As used herein, the terms "or" and "and/or" are utilized to describe multiple components in combination or exclusive of one another. For example, "x, y, and/or z" can refer to "x" alone, "y" alone, "z" alone, "x, y, and z," "(x and y) or z," "x or (y and z)," or "x or y or z." It is specifically contemplated that x, y, or z may be specifically excluded from an embodiment.

Throughout this application, the term "about" is used according to its plain and ordinary meaning in the area of cell and molecular biology to indicate that a value includes the standard deviation of error for the device or method being employed to determine the value. "About" when referring to a measurable value, such as an amount of a composition, a dose, a time, a temperature, and the like, is meant to encompass variations of ± 20%, ± 10%, ± 5%, ± 1%, ± 0.5%, or even ± 0.1% of the specified amount.

The term "adeno-associated virus" (AAV) in the context of the present disclosure includes without limitation AAV type 1, AAV type 2, AAV type 3 (including types 3A and 3B), AAV type 4, AAV type 5, AAV type 6, AAV type 7, AAV type 8, AAV type 9, AAV type 10, AAV type 11, avian AAV, bovine AAV, canine AAV, equine AAV, and ovine AAV and any other AAV now known or later discovered, or engineered from the above-mentioned AAV. The AAV may be an AAV-PHP.eB, AAV-PHP.B, AAV-PHP.S, AAV-PHP.B4, AAV-PHP.B5, AAV-PHP.N, AAV-CAP-B1, AAV-CAP-B10, or AAV-CAP-B22, for example.

The words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The compositions and methods for their use can "comprise," "consist essentially of," or "consist of" any of the ingredients or steps disclosed throughout the specification. Compositions and methods "consisting essentially of" any of the ingredients or steps disclosed limits the scope of the claim to the specified materials or steps which do not materially affect the basic and novel characteristic of the disclosure.

As used herein, the term "therapeutically effective amount" is synonymous with "effective amount", "therapeutically effective dose", and/or "effective dose" and refers to the amount of compound that will elicit the biological, cosmetic or clinical response being sought by the practitioner in an individual in need thereof. The appropriate effective amount to be administered for a particular application of the disclosed methods can be determined by those skilled in the art, using the guidance provided herein. For example, an effective amount can be extrapolated from in vitro and in vivo assays as described in the present specification. One skilled in the art will recognize that the condition of the individual can be monitored throughout the course of therapy and that the effective amount of a compound or composition disclosed herein that is administered can be adjusted accordingly.

As used herein, "transduction" of a cell by a viral particle, including any AAV viral particle encompassed herein, means entry of the particle into the cell and transfer of genetic material into the cell by the incorporation of nucleic acid into the virus vector and subsequent transfer into the cell via the virus vector.

Reference throughout this specification to "one embodiment," "an embodiment," "a particular embodiment," "a related embodiment," "a certain embodiment," "an additional embodiment," or "a further embodiment" or combinations thereof means that a particular feature, structure or characteristic described in connection with the embodiment is included in at least one embodiment of the present invention. Thus, the appearances of the foregoing phrases in various places throughout this specification are not necessarily all referring to the same embodiment. Furthermore, the particular features, structures, or characteristics may be combined in any suitable manner in one or more embodiments.

As used herein, the term "individual" or "subject" generally refers to an individual in need of a therapy. The individual can be a mammal, such as a human, dog, cat, horse, pig or rodent. The individual can be a that has or is suspected of having or at risk for having a disease or medical condition, including related to encephalopathy of any kind. For individuals having or suspected of having a medical condition directly or indirectly associated with encephalopathies, the medical condition may be of one or more types. The individual may have a disease or be suspected of having the disease. The individual may be asymptomatic. The individual may be of any gender.

As used herein, the term "regulatory sequence" refers to any segment of a nucleic acid that is capable of increasing or decreasing the expression of a gene. The regulatory sequence may be proximate to the gene it is regulating, such as, for example, a nucleic acid with less than 100, less than 50, less than 20, less than 10, or less than 5 nucleotides separating the 5' end or 3' end of the gene from the 5' end or 3' end of the regulatory sequence. The regulatory system may be distant to the gene it is regulating, such as, for example, a nucleic acid with 100 or more, 200 or more, 300 or more, 400 or more, 500 or more, 600 or more, 700 or more, 800 or more, 900 or more, 1000 or more nucleotides separating the 5' end or 3' end of the gene from the 5' end or 3' end of the regulatory sequence. A regulatory sequence may comprise a promoter and/or an enhancer. A regulatory sequence may or may not be on the same nucleic acid molecule of the gene the regulatory sequence is regulating. A regulatory sequence, including any regulatory sequence, promoter, or enhancer used herein, may be named by the gene in which it regulates. For example, the STXBP1 promoter used in compositions of the disclosure may comprise the regulatory sequence that regulates the human STXBP1 gene. In some embodiments, a heterologous promoter with respect to the STXBP1 gene may be employed instead.

As used herein, the terms "treatment," "treat," or "treating" refers to intervention in an attempt to alter the natural course of the individual or cell being treated, and may be performed either for prophylaxis or during the course of pathology of a disease or condition. Treatment may serve to accomplish one or more of various desired outcomes, including, for example, preventing occurrence or recurrence of disease, alleviation of symptoms, and diminishment of any direct or indirect pathological consequences of the disease, lowering the rate of disease progression, amelioration or palliation of the disease state, and remission or improved prognosis.

### II. Compositions for Restoring STXBPI

Embodiments of the disclosure concern compositions, including gene therapies, viral particles, and nucleic acids, that are useful for restoring STXBP1 in individuals with any deficiency, including missense mutations, nonsense mutations, deletions, inversions, insertions, duplications, frameshift mutations, repeat expansions, haploinsufficiencies, or a combination thereof in the *STXBP1* gene. In some embodiments, the composition comprises a viral particle. The viral particle may comprise one or more nucleic acids. The nucleic acids may encode for an *STXBP1* gene product. A *"STXBPI* gene product" describes a polypeptide generated from transcription and translation of a *STXBP1* gene. A *STXBP1* gene product may be of any STXBP1 isoform (e.g., isoform a, isoform b, isoform c, isoform d, isoform e, isoform f, isoform g, or isoform h). The nucleic acid that encodes an *STXBP1* gene product may be a *STXBP1* gene from any organism, including for example, a worm, a fruit fly, a mouse, a rat, any non-human primate, and a human. The nucleic acid that encodes an STXBP1 gene product may be an *STXBP1* gene with one or more silent mutations. In some embodiments, the nucleic acid encodes for isoform a of STXBP1 (also known as STXBP1a). In some embodiments, the nucleic acid encodes for isoform b of STXBP1 (also known as STXBP1b). In some embodiments, the nucleic acid encodes for isoform c of STXBP1 (also known as STXBP1c). In some embodiments, the nucleic acid encodes for isoform d of STXBP1 (also known as STXBP1d).

In some embodiments, the viral particle comprises a nucleic acid that encodes for an amino acid sequence having, having at least, or having at most 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with SEQ ID NO:1, or any range or value derivable therein. In certain embodiments, the viral particle comprises a nucleic acid that encodes for the STXBP1 amino acid sequence of SEQ ID NO:1 (isoform a).

In some embodiments, the viral particle comprises a nucleic acid that encodes for an amino acid sequence having, having at least, or having at most 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with SEQ ID NO:2, or any range or value derivable therein. In certain embodiments, the viral particle comprises a nucleic acid that encodes for the STXBP1 amino acid sequence of SEQ ID NO:2 (isoform b).

In some embodiments, the viral particle comprises a nucleic acid that encodes for an amino acid sequence having, having at least, or having at most 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with SEQ ID NO:3, or any range or value derivable therein. In certain embodiments, the viral particle comprises a nucleic acid that encodes for the STXBP1 amino acid sequence of SEQ ID NO:3 (isoform c).

In some embodiments, the viral particle comprises a nucleic acid that encodes for an amino acid sequence having, having at least, or having at most 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with SEQ ID NO:4, or any range or value derivable therein. In certain embodiments, the viral particle comprises a nucleic acid that encodes for the STXBP1 amino acid sequence of SEQ ID NO:4 (isoform d).

Particular sequences of STXBP1 isoform a, STXBP1 isoform b, STXBP1 isoform c, and STXBP1 isoform d are provided below.

1 

61 

121 

181 

241 

301 

361 

421 

481 

541 

601 

(SEQ ID NO:1, STXBP1 isoform a)

1 mapiglkavv gekimhdvik kvkkkgewkv lvvdqlsmrm lsscckmtdi mtegitived

61 inkrreplps leavylitps eksvhslisd fkdpptakyr aahvfftdsc pdalfnelvk

121 sraakviktl teiniaflpy esqvysldsa dsfqsfysph kaqmknpile rlaeqiatlc

181 atlkeypavr yrgeykdnal laqliqdkld aykaddptmg egpdkarsql lildrgfdps

241 spvlheltfq amsydllpie ndvykyetsg igearvkevl ldedddlwia lrhkhiaevs

301 qevtrslkdf ssskrmntge kttmrdlsqm lkkmpqyqke lskysthlhl aedcmkhyqg

361 tvdklcrveq dlamgtdaeg ekikdpmrai vpilldanvs tydkiriill yiflkngite

421 enlnkliqha qippedseii tnmahlgvpi vtdstlrrrs kperkerise qtyqlsrwtp

481 iikdimedti edkldtkhyp yistrssasf sttavsaryg hwhknkapge yrsgprliif

541 ilggvslnem rcayevtqan gkwevligst hiltpqklld tlkklnktde eiss

(SEQ ID NO:2, STXBP1 isoform b).

1 mapiglkavv gekimhdvik kvkkkgewkv lvvdqlsmrm lsscckmtdi mtegitived

61 inkrreplps leavylitps eksvhslisd fkdpptakyr aahvfftdya lfnelvksra

121 akviktltei niaflpyesq vysldsadsf qsfysphkaq mknpilerla eqiatlcatl

181 keypavryrg eykdnallaq liqdkldayk addptmgegp dkarsqllil drgfdpsspv

241 lheltfqams ydllpiendv ykyetsgige arvkevllde dddlwialrh khiaevsqev

301 trslkdfsss krmntgektt mrdlsqmlkk mpqyqkelsk ysthlhlaed cmkhyqgtvd

361 klcrveqdla mgtdaegeki kdpmraivpi lldanvstyd kiriillyif lkngiteenl

421 nkliqhaqip pedseiitnm ahlgvpivtd stlrrrskpe rkeriseqty qlsrwtpiik

481 dimedtiedk ldtkhypyis trssasfstt avsaryghwh knkapgeyrs gprliifilg

541 gvslnemrca yevtqangkw evligsthil tpqklldtlk klnktdeeis s

1   mhdvikkvkk kgewkvlvvd qlsmrmlssc ckmtdimteg itivedinkr replpsleav

61  ylitpseksv hslisdfkdp ptakyraahv fftdscpdal fnelvksraa kviktltein

121 iaflpyesqv ysldsadsfq sfysphkaqm knpilerlae qiatlcatlk eypavryrge

181 ykdnallaql iqdkldayka ddptmgegpd karsqllild rgfdpsspvl heltfqamsy

241 dllpiendvy kyetsgigea rvkevllded ddlwialrhk hiaevsqevt rslkdfsssk

301 rmntgekttm rdlsqmlkkm pqyqkelsky sthlhlaedc mkhyqgtvdk lcrveqdlam

361 gtdaegekik dpmraivpil ldanvstydk iriillyifl kngiteenln kliqhaqipp

421 edseiitnma hlgvpivtds tlrrrskper keriseqtyq lsrwtpiikd imedtiedkl

481 dtkhypyist rssasfstta vsaryghwhk nkapgeyrsg prliifilgg vslnemrcay

541 evtqangkwe vligsthilt ptkflmdlrh pdfressrvs fedqaptme

(SEQ ID NO:4, STXBP1 isoform d).

In certain embodiments, the nucleic acid encodes for part or all of SEQ ID NO:1, SEQ ID NO:2, SEQ ID NO:3, or SEQ ID NO:4. In some embodiments, the viral particle comprises a sequence that encodes for an amino acid sequence that is at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identical to SEQ ID NO:1, SEQ ID NO:2, SEQ ID NO:3, or SEQ ID NO:4. In some embodiments, the viral particle comprises a nucleic acid sequence encoding SEQ ID NO:1, SEQ ID NO:2, SEQ ID NO:3, or SEQ ID NO:4, wherein 1, 2 or fewer, 3 or fewer, 4 or fewer, 5 or fewer, 6 or fewer, 7 or fewer, 8 or fewer, 9 or fewer, 10 or fewer, 12 or fewer, 15 or fewer, 20 or fewer, 25 or fewer, 30 or fewer, 40 or fewer, or 50 or fewer of the codons within SEQ ID NO:1, SEQ ID NO:2, SEQ ID NO:3, or SEQ ID NO:4 is substituted with another codon, optionally comprising a conservative amino acid substitution or silent mutation, and/or are deleted and/or an insertion (including 5' and/or 3' extensions) of 1, 2 or fewer, 3 or fewer, 4 or fewer, 5 or fewer, 6 or fewer, 7 or fewer, 8 or fewer, 9 or fewer, 10 or fewer, 12 or fewer, 15 or fewer, 20 or fewer, 25 or fewer, 30 or fewer, 40 or fewer, or 50 or fewer codons or any combination of substitutions, deletions and/or insertions, wherein the substitutions, deletions and/or insertions do not unduly impair the structure and/or function of STXBP1.

Conservative amino acid substitutions are known in the art. In particular embodiments, a conservative amino acid substitution includes substitutions within one or more of the following groups: glycine, alanine; valine, isoleucine, leucine; aspartic acid, glutamic acid; asparagine, glutamine; serine, threonine; lysine, arginine; and/or phenylalanine, tyrosine.

In certain embodiments, the viral particle comprises one or more nucleic acid sequences encoding a regulatory sequence. The regulatory sequence may be a promoter and/or enhancer for genes specifically expressed in cells of the central and/or peripheral nervous system, including glutamatergic neurons, GABAergic neurons, glycinergic neurons, cholinergic neurons, dopaminergic neurons, serotonergic neurons, adrenergic neurons, noradrenergic neurons, parvalbumin neurons and glial cells. It is specifically contemplated that promoters and/or enhancers specific for one or more of the preceding cell types may be excluded from certain embodiments of the disclosure. In some embodiments, the viral particle does not comprise a regulatory sequence that is specific for parvalbumin neurons. In some embodiments, the regulatory sequence comprises a promoter selected from the group consisting of CAG, CBA, EF1a, UbiC, CMV, hSyn1, MeCP2, NSE, BM88, Vglutl, Vglut2, CaMKII, Vgat, DLX5/6, mDlx, NR2E1, and a combination thereof. One of more of the preceding promoters may be excluded from embodiments of the disclosure.

A variety of promoter/enhancer elements may be used depending on the level and tissue-specific expression desired. The promoter/enhancer may be constitutive or inducible, depending on the pattern of expression desired. The promoter/enhancer may be native or foreign and can be a natural or a synthetic sequence. By foreign, it is intended that the transcriptional initiation region is not found in the wild-type host into which the transcriptional initiation region is introduced.

Promoter/enhancer elements can be native to the target cell or subject to be treated and/or native to the heterologous nucleic acid sequence. The promoter/enhancer element is generally chosen so that it will function in the target cell(s) of interest. In representative embodiments, the promoter/enhancer element is a mammalian promoter/enhancer element. The promoter/enhance element may be constitutive or inducible.

In some embodiments, the viral particle comprises an AAV particle, such as any AAV particle encompassed herein. The viral particle may be an AAV-PHP.eB particle, AAV-PHP.B particle, AAV-PHP.S particle, AAV-PHP.B4 particle, AAV-PHP.B5 particle, AAV-PHP.N particle, AAV-CAP-B1 particle, AAV-CAP-B10 particle, AAV-CAP-B22 particle, AAV type 1 particle, AAV type 5 particle, AAV type 8 particle, or AAV type 9 particle. In some embodiments, the viral particle is an AAV-PHP.eB particle.

In some embodiments, the viral particle is a particle described in one or more of PCT Patent Application Publications WO2020206189, WO2020028751, WO2020168145, WO2020077165, WO2021025995, WO2017197355, WO2018022905, WO201700671, WO2017218842, WO2016154344, WO2017192750; WO2016081811, WO2019222329 and WO2019028306, all of which are incorporated by reference herein in their entirety.

In some embodiments, the composition comprises at least one nucleic acid molecule that encodes for one or more gene products capable of generating one or more viral particles, including any viral particle encompassed herein. The nucleic acid(s) may comprise one or more plasmids, including any viral plasmids. The plasmid(s) may be an AAV plasmid, including an AAV-PHP.eB, AAV-PHP.B, AAV-PHP.S, AAV-PHP.B4, AAV-PHP.B5, AAV-PHP.N, AAV-CAP-B1, AAV-CAP-B10, AAV-CAP-B22, AAV type 1, AAV type 5, AAV type 8, or AAV type 9 plasmid. The plasmid(s) may be an AAV plasmid as described in one or more of PCT Patent Application Publications WO2020206189, WO2020028751, WO2020168145, WO2020077165, WO2021025995, WO2017197355, WO2018022905, WO201700671, WO2017218842, WO2016154344, WO2017192750; WO2016081811, WO2019222329 and WO2019028306, all of which are incorporated by reference herein in their entirety. The nucleic acid may comprise a sequence that encodes for a transgene. The transgene may encode any gene product encompassed herein, including any STXBP1 gene product encompassed herein. The nucleic acid molecules may comprise any regulatory sequence encompassed herein.

A plasmid of the disclosure may be an AAV plasmid comprising one or more of the following amino acid sequences: SEQ ID NOs: 15-16 of PCT patent application publication WO2020206189, SEQ ID NOs: 4-14326 and 42973-42999 of PCT patent application publication WO2020028751; SEQ ID NO: 1829 of PCT patent application publication WO2020077165; SEQ ID NO: 43073-43341 of PCT patent application publication WO2021025995, SEQ ID NOs: 14-41 of PCT patent application publication WO2017197355, SEQ ID NOs: 47-142 of PCT patent application publication WO2018022905, SEQ ID NO: 37 of PCT patent application publication WO201700671, SEQ ID NOs: 3-78 of PCT patent application publications WO2017218842 and WO2016154344, and SEQ ID NOs: 8-14 of PCT patent application publication WO2017192750. A plasmid of the disclosure may be an AAV plasmid comprising one or more of the following nucleotide sequences: SEQ ID NOs: 1-43 of PCT patent application publication WO2016081811, SEQ ID NOs: 1824-1825 of PCT patent application publication WO2019222329, and SEQ ID NOs: 1809-1810 of PCT patent application publication WO2019028306. All of the preceding PCT patent application publications are incorporated by reference herein in their entirety.

Certain embodiments of the disclosure concern methods of producing viral particles. In some embodiments, the method comprises providing to a cell in vitro, (a) a template comprising (i) a nucleic acid encoding for an STXBP1 gene product, and (ii) packaging signal sequences sufficient for the encapsidation of an AAV template into virus particles (e.g., one or more (e.g., two) terminal repeats, such as AAV terminal repeats), and (b) AAV sequences sufficient for replication and encapsidation of the template into viral particles (e.g., the AAV rep and AAV cap sequences encoding an AAV capsid). The template and AAV replication and capsid sequences are provided under conditions such that recombinant virus particles comprising the template packaged within the capsid are produced in the cell. The method can further comprise the step of collecting the virus particles from the cell. Virus particles may be collected from the medium and/or by lysing the cells.

The cell is typically a cell that is permissive for AAV viral replication. Any suitable cell known in the art may be employed, such as mammalian cells. Also suitable are trans-complementing packaging cell lines that provide functions deleted from a replication-defective helper virus, e.g., 293 cells or other E1a trans-complementing cells.

In particular embodiments, the present disclosure provides a pharmaceutical composition comprising a vector, including a virus vector, of the disclosure in a pharmaceutically acceptable carrier and, optionally, other medicinal agents, pharmaceutical agents, stabilizing agents, buffers, carriers, adjuvants, diluents, etc. For injection, the carrier will typically be a liquid. For other methods of administration, the carrier may be either solid or liquid. For inhalation administration, the carrier will be respirable, and will preferably be in solid or liquid particulate form.

### III. Methods for Treating Encephalopathies by Restoring STXBP1

Certain embodiments of the disclosure concern the treatment of an individual with an encephalopathy. The encephalopathy may be an epileptic encephalopathy, such as an early infantile epileptic encephalopathy. In some embodiments, the individual has Ohtahara syndrome, West syndrome, Lennox-Gastaut syndrome, Dravet syndrome, or Rett syndrome with mutated *STXBP1.* In some embodiments, the encephalopathy is STXBP1 encephalopathy or early infantile epileptic encephalopathy type 4. In some embodiments, the encephalopathy is STXBP1 encephalopathy. The encephalopathy may be caused by, or associated with, one or more missense mutations, nonsense mutations, deletions, inversions, insertions, duplications, frameshift mutations, repeat expansions, haploinsufficiencies, or a combination thereof in a gene, including *STXBP1.* The encephalopathy may be caused by, or associated with, one or more mutations in a gene, including *STXBP1.*

In some embodiments, an individual is treated by a method comprising administering a therapeutically effective amount of one or more compositions encompassed herein, including any viral particle herein, to the individual. The composition may increase the level of a heterologous transgene, including STXBP1, in the individual, including in cells of the individual. In some embodiments, the composition administered to the individual restores the level of STXBP1 to a level found in a control individual (i.e. an individual that does not have an STXBP1 haploinsufficiency and/or mutation). In some embodiments, the compositions restore cognitive abilities in the individual. In some embodiments, the compositions reduce the number and/or severity of seizures in the individual. In some embodiments, the compositions restore motor functions in the individual. In some embodiments, the compositions restore psychiatric functions in the individual.

Effective dosages of the viral particles to be administered to a subject will depend upon the mode of administration, the disease or condition to be treated, the individual subject's condition, the particular virus vector, and the nucleic acid to be delivered, and can be determined in a routine manner. Examples of effective doses for achieving therapeutic effects include virus titers of at least about 10⁵, 10⁶, 10⁷, 10⁸, 10⁹, 10¹⁰, 10¹¹, 10¹², 10¹¹, 10¹⁴, 10¹⁵ transducing units or more.

Example modes of administration include oral, rectal, transmucosal, topical, intranasal, inhalation (e.g., via an aerosol), buccal (e.g., sublingual), vaginal, intrathecal, intraocular, transdermal, in utero (or in ovo), parenteral (e.g., intravenous, subcutaneous, intradermal, intramuscular (including administration to skeletal, diaphragm and/or cardiac muscle), intradermal, intrapleural, intracerebral, intracerebroventricular, intraparenchymal, and intraarticular), topical (e.g., to both skin and mucosal surfaces, including airway surfaces, and transdermal administration), intro-lymphatic, and the like, as well as direct tissue or organ injection (e.g., to brain, liver, muscle, etc.). The most suitable route in any given case will depend on the nature and severity of the condition being treated and on the nature of the particular vector and/or viral particle that is being used.

In some embodiments, the viral particle is administered directly to the CNS, e.g., the brain or the spinal cord. Direct administration can result in high specificity of transduction of CNS cells, e.g., wherein at least 50%, 55%, 60%, 65%, 70%, 75%, 80%, 81%, 82%, 83%, 84%, 85%, 86%, 87%, 88%, 90%, 91%, 92%, 93%, 94%, 95%, 96%, 97%, 98%, 99%, or more of the transduced cells are CNS cells. Any method known in the art to administer vectors directly to the CNS can be used. The vector may be introduced into the spinal cord, brainstem (medulla oblongata, pons), midbrain (hypothalamus, thalamus, epithalamus, pituitary gland, substantia nigra, pineal gland), cerebellum, telencephalon (corpus striatum, cerebrum including the occipital, temporal, parietal and frontal lobes, cortex, basal ganglia, hippocampus and amygdala), limbic system, neocortex, corpus striatum, cerebrum, and inferior colliculus. The vector may also be administered to different regions of the eye such as the retina, cornea or optic nerve. The vector may be delivered into the cerebrospinal fluid (e.g., by lumbar puncture) for more disperse administration of the vector.

The delivery vector may be administered to the desired region(s) of the CNS by any route known in the art, including but not limited to, intrathecal, intracerebral, intraventricular, intranasal, intra-aural, intra-ocular (e.g., intra-vitreous, sub-retinal, anterior chamber) and periocular (e.g., sub-Tenon's region) delivery or any combination thereof.

Typically, the viral vector will be administered in a liquid formulation by direct injection to the desired region or compartment in the CNS. In some embodiments, the vector can be delivered via a reservoir and/or pump. In other embodiments, the vector may be provided by topical application to the desired region or by intra-nasal administration of an aerosol formulation. Administration to the eye or into the ear, may be by topical application of liquid droplets. As a further alternative, the vector may be administered as a solid, slow-release formulation.

Embodiments of the disclosure include at least three ways to deliver the AAV particles. In some embodiments, one can inject the AAV particles directly into the brain tissues. In some embodiments, one can deliver the particles into cerebrospinal fluid (CSF), such as by injection into the ventricle or lumbar intrathecal space. In some embodiments, one can deliver the particles systemically, such as by injection into a blood vessel, and then let the AAV particles cross the blood brain barrier (BBB). In particular embodiments, one or more AAV particles of the disclosure has the ability to cross the blood brain barrier (BBB). In embodiments wherein any AAV particle is considered to have very weak or no ability to cross BBB, one can inject the AAV particles directly into the brain tissues, such as by intraparenchymal injection.

### IV. Polypeptides

As used herein, a "protein" or "polypeptide" refers to a molecule comprising at least five amino acid residues. As used herein, the term "wild-type" refers to the endogenous version of a molecule that occurs naturally in an organism. In some embodiments, wild-type versions of a protein or polypeptide are employed, however, in many embodiments of the disclosure, a modified protein or polypeptide is employed. The terms described above may be used interchangeably. A "modified protein" or "modified polypeptide" or a "variant" refers to a protein or polypeptide whose chemical structure, particularly its amino acid sequence, is altered with respect to the wild-type protein or polypeptide. In some embodiments, a modified/variant protein or polypeptide has at least one modified activity or function (recognizing that proteins or polypeptides may have multiple activities or functions). It is specifically contemplated that a modified/variant protein or polypeptide may be altered with respect to one activity or function yet retain a wild-type activity or function in other respects.

Where a protein is specifically mentioned herein, it is in general a reference to a native (wild-type) or recombinant (modified) protein or, optionally, a protein in which any signal sequence has been removed. The protein may be isolated directly from the organism of which it is native, produced by recombinant DNA/exogenous expression methods, or produced by solid-phase peptide synthesis (SPPS) or other in vitro methods. In particular embodiments, there are isolated nucleic acid segments and recombinant vectors incorporating nucleic acid sequences that encode a polypeptide. The term "recombinant" may be used in conjunction with a polypeptide or the name of a specific polypeptide, and this generally refers to a polypeptide produced from a nucleic acid molecule that has been manipulated in vitro or that is a replication product of such a molecule.

In certain embodiments the size of a protein or polypeptide (wild-type or modified) may comprise, but is not limited to, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 275, 300, 325, 350, 375, 400, 425, 450, 475, 500, 525, 550, 575, 600, 625, 650, 675, 700, 725, 750, 775, 800, 825, 850, 875, 900, 925, 950, 975, 1000, 1100, 1200, 1300, 1400, 1500, 1750, 2000, 2250, 2500 amino acid residues or greater, and any range derivable therein, or derivative of a corresponding amino sequence described or referenced herein. It is contemplated that polypeptides may be mutated by truncation, rendering them shorter than their corresponding wild-type form, also, they might be altered by fusing or conjugating a heterologous protein or polypeptide sequence with a particular function (e.g., for targeting or localization, for purification purposes, etc.). As used herein, the term "domain" refers to any distinct functional or structural unit of a protein or polypeptide, and generally refers to a sequence of amino acids with a structure or function recognizable by one skilled in the art.

The nucleotide as well as the protein, polypeptide, and peptide sequences for various genes have been previously disclosed, and may be found in the recognized computerized databases. Two commonly used databases are the National Center for Biotechnology Information's Genbank and GenPept databases (on the World Wide Web at ncbi.nlm.nih.gov/) and The Universal Protein Resource (UniProt; on the World Wide Web at uniprot.org). The coding regions for these genes may be amplified and/or expressed using the techniques disclosed herein or as would be known to those of ordinary skill in the art.

It is contemplated that in compositions of the disclosure, there is between about 0.001 mg and about 10 mg of total polypeptide, peptide, and/or protein per ml. The concentration of protein in a composition can be about, at least about or at most about 0.001, 0.010, 0.050, 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0 mg/ml or more (or any range derivable therein).

### V. Vectors

STXBP1 gene products may be delivered to recipient cells (e.g., cells of the peripheral and/or central nervous system) by any suitable vector, including by a viral vector or by a non-viral vector. Examples of viral vectors include at least retroviral, lentiviral, adenoviral, or adeno-associated viral vectors. Examples of non-viral vectors include at least plasmids, transposons, lipids, nanoparticles, and so forth. One of skill in the art would be well-equipped to construct a vector through standard recombinant techniques (see, for example, Sambrook *et al.,* 2001 and Ausubel *et al.,* 1996, both incorporated herein by reference) for the expression of the antigen receptors of the present disclosure.

### VI. Pharmaceutical Compositions and Delivery

The disclosure concerns compositions comprising, consisting essentially of, or consisting of an adeno-associated virus (AAV) particle comprising a nucleic acid comprising a sequence encoding part or all of an STXBP1 gene product, including wherein the AAV particle comprises one or more elements that imparts activity of the particle to transduce cells of the central and/or peripheral nervous system of an individual in need thereof. In cases wherein the nucleic acid encodes part of the STXBP1 gene product, the part will be functional in treating a medical condition associated with defective STXBP1, as addressed elsewhere herein.

In particular embodiments, any composition encompassed herein comprises a pharmaceutically acceptable (e.g., physiologically acceptable) carrier. When the composition consists essentially of the inventive particle and a pharmaceutically acceptable carrier, additional components can be included that do not materially affect the composition (*e.g*., adjuvants, buffers, stabilizers, anti-inflammatory agents, solubilizers, preservatives, *etc*.)*.* When the composition consists of the inventive particle and the pharmaceutically acceptable carrier, the composition does not comprise any additional components. Any suitable carrier can be used within the context of the disclosure, and such carriers are well known in the art. The choice of carrier will be determined, in part, by the particular site to which the composition may be administered and the particular method used to administer the composition. The composition optionally can be sterile with the exception of the composition components described herein. The composition can be frozen or lyophilized for storage and reconstituted in a suitable sterile carrier prior to use. The compositions can be generated in accordance with conventional techniques described in, e.g., Remington: The Science and Practice of Pharmacy, 21st Edition, Lippincott Williams & Wilkins, Philadelphia, Pa. (2001).

Suitable formulations for the composition include aqueous and non-aqueous solutions, isotonic sterile solutions, which can contain anti-oxidants, buffers, and bacteriostats, and aqueous and non-aqueous sterile suspensions that can include suspending agents, solubilizers, thickening agents, stabilizers, and preservatives. The formulations can be presented in unit-dose or multidose sealed containers, such as ampules and vials, and can be stored in a freeze-dried (lyophilized) condition requiring only the addition of the sterile liquid carrier, for example, water, immediately prior to use. Extemporaneous solutions and suspensions can be prepared from sterile powders, granules, and tablets of the kind previously described. In one embodiment, the carrier is a buffered saline solution. In one embodiment, the inventive particle is administered in a composition formulated to protect the composition and its contents from damage prior to administration. For example, the composition can be formulated to reduce damage from devices used to prepare, store, or administer the particle, such as glassware, syringes, or needles. The composition can be formulated to decrease the light sensitivity and/or temperature sensitivity of the nucleic acid. To this end, the composition may comprise a pharmaceutically acceptable liquid carrier, such as, for example, those described above, and a stabilizing agent selected from the group consisting of polysorbate 80, L-arginine, polyvinylpyrrolidone, trehalose, and combinations thereof. Use of such a composition extends the shelf life of the gene transfer vector, facilitate administration, and increase the efficiency of the inventive method. Formulations for gene transfer vector-containing compositions are further described in, for example, Wright et al., Curr. Opin. Drug Discov. Devel., 6(2): 174-178 (2003) and Wright et al., Molecular Therapy, 12: 171-178 (2005))

The composition also can be formulated to enhance transduction efficiency. In addition, one of ordinary skill in the art will appreciate that the inventive gene transfer composition can be present in a composition with other therapeutic or biologically-active agents. For example, factors that control inflammation, such as ibuprofen or steroids, can be part of the composition to reduce swelling and inflammation associated with in vivo administration of the gene transfer composition. Immune system stimulators or adjuvants, *e.g.*, interleukins, lipopolysaccharide, and double-stranded RNA, can be administered to enhance or modify the anti-tau immune response. Antibiotics, *i.e.,* microbicides and fungicides, can be present to treat existing infection and/or reduce the risk of future infection, such as infection associated with gene transfer procedures.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

In certain embodiments, a formulation of the present disclosure comprises a biocompatible polymer selected from the group consisting of polyamides, polycarbonates, polyalkylenes, polymers of acrylic and methacrylic esters, polyvinyl polymers, polyglycolides, polysiloxanes, polyurethanes and co-polymers thereof, celluloses, polypropylene, polyethylenes, polystyrene, polymers of lactic acid and glycolic acid, polyanhydrides, poly(ortho)esters, poly(butic acid), poly(valeric acid), poly(lactide-co-caprolactone), polysaccharides, proteins, polyhyaluronic acids, polycyanoacrylates, and blends, mixtures, or copolymers thereof.

The composition can be administered in or on a device (such as a mechanical reservoir) that allows controlled or sustained release, such as a sponge, biocompatible meshwork, mechanical reservoir, or mechanical implant. Implants, devices, such as an implantable device, *e.g.,* a mechanical reservoir or an implant or a device comprised of a polymeric composition, are particularly useful for administration of the inventive gene transfer vector. The composition also can be administered in the form of sustained-release formulations (see, e.g., U.S. Pat. No. 5,378,475) comprising, for example, gel foam, hyaluronic acid, gelatin, chondroitin sulfate, a polyphosphoester, such as bis-2-hydroxyethyl-terephthalate (BHET), and/or a polylactic-glycolic acid.

Delivery of the compositions comprising the inventive gene transfer compositions may be intracerebral (including but not limited to intracerebroventricular, intraparenchymal, intraventricular, or intracisternal), intrathecal (including but not limited to lumbar or cisterna magna), or systemic, including but not limited to intravenous, or any combination thereof, using devices known in the art. Delivery may also be via surgical implantation of an implanted device.

The dose of the gene transfer composition administered to a mammal will depend on a number of factors, including the size (mass) of the mammal, the extent of any side-effects, the particular route of administration, and the like. In one embodiment, the inventive method comprises administering a "therapeutically effective amount" of the composition comprising the inventive gene transfer composition described herein. A "therapeutically effective amount" refers to an amount effective, at dosages and for periods of time necessary, to achieve a desired therapeutic result. The therapeutically effective amount may vary according to factors such as the extent of the disease, age, gender, and/or weight of the individual, and the ability of the gene transfer composition to elicit a desired response in the individual. The dose of gene transfer composition required to achieve a particular therapeutic effect may be determined by standard means. An example of a range of doses includes 1011 vg/kg to 1014 vg/kg.

In one embodiment of the disclosure, the composition is administered once to the mammal. However, in certain cases, it may be appropriate to administer the composition multiple times during a therapeutic period to ensure sufficient exposure of cells to the composition. For example, the composition may be administered to the mammal two or more times (e.g., 2, 3, 4, 5, 6, 6, 8, 9, or 10 or more times) during a therapeutic period.

The present disclosure provides pharmaceutically acceptable compositions that comprise a therapeutically-effective amount of composition comprising a nucleic acid sequence that encodes a functional STXBP1.

### VII. Subjects

The subject (or individual or patient) that is the recipient of methods and compositions of the disclosure may be any subject with a defective *STXBP1* for any reason. The subject may be any animal, including a human and non-human animal. Non-human animals include all vertebrates, e.g., mammals and non-mammals, such as non-human primates, sheep, dogs, cats, cows, horses, chickens, amphibians, and reptiles, although mammals are envisioned as subjects, such as non-human primates, sheep, dogs, cats, cows and horses. The subject may also be livestock such as, cattle, swine, sheep, poultry, and horses, or pets, such as dogs and cats.

Particular subjects include human subjects suffering from or at risk for the medical diseases and conditions described herein. The subject may be generally diagnosed with the condition of the disclosure by skilled artisans, such as a medical practitioner. In some cases, the methods of the disclosure include steps of determining the presence of defective *STXBP1* in a suitable sample from the subject.

The methods of the disclosure described herein can be employed for subjects of any species, gender, sex, age, ethnic population, and/or genotype. Accordingly, the term subject includes males and females, and it includes elderly, elderly-to-adult transition age subjects, adults, adult-to-pre-adult transition age subjects, and pre-adults, including adolescents, children, and infants. Examples of human ethnic populations include Caucasians, Asians, Hispanics, Africans, African Americans, Native Americans, Semites, and Pacific Islanders.

The term subject also includes subjects of any genotype or phenotype as long as they are in need of the disclosed methods and compositions, as described above. In addition, the subject can have the genotype or phenotype for any hair color, eye color, skin color or any combination thereof. The term subject includes a subject of any body height, body weight, or any organ or body part size or shape.

### VIII. Kits of the Disclosure

Any of the viral and/or non-viral compositions described herein or similar thereto may be comprised in a kit. In a non-limiting example, one or more reagents for use in methods for preparing viral particles may be comprised in a kit. Such reagents may include cells, vectors, one or more growth factors, one or more costimulatory factors, media, enzymes, buffers, nucleotides, salts, primers, compounds, and so forth. The kit components are provided in suitable container means.

Some components of the kits may be packaged either in aqueous media or in lyophilized form. The container means of the kits will generally include at least one vial, test tube, flask, bottle, syringe or other container means, into which a component may be placed, and preferably, suitably aliquoted. Where there are more than one component in the kit, the kit also will generally contain a second, third or other additional container into which the additional components may be separately placed. However, various combinations of components may be comprised in a vial. The kits of the present disclosure also will typically include a means for containing the components in close confinement for commercial sale. Such containers may include injection or blow molded plastic containers into which the desired vials are retained.

When the components of the kit are provided in one and/or more liquid solutions, the liquid solution is an aqueous solution, with a sterile aqueous solution being particularly useful. In some cases, the container means may itself be a syringe, pipette, and/or other such like apparatus, or may be a substrate with multiple compartments for a desired reaction.

Some components of the kit may be provided as dried powder(s). When reagents and/or components are provided as a dry powder, the powder can be reconstituted by the addition of a suitable solvent. It is envisioned that the solvent may also be provided in another container means. The kits may also comprise a second container means for containing a sterile acceptable buffer and/or other diluent.

In specific embodiments, reagents and materials include primers for amplifying desired sequences, nucleotides, suitable buffers or buffer reagents, salt, and so forth, and in some cases the reagents include apparatus or reagents for isolation of a particular desired cell(s).

### EXAMPLES

The following examples are included to demonstrate particular embodiments of the invention. It should be appreciated by those of skill in the art that the techniques disclosed in the examples that follow represent techniques discovered by the inventors to function well in the practice of the methods of the disclosure, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the disclosure.

### EXAMPLE 1

### STXBP1 HAPLOINSUFFICIENCY CAUSES STXBP1 ENCEPHALOPATHY

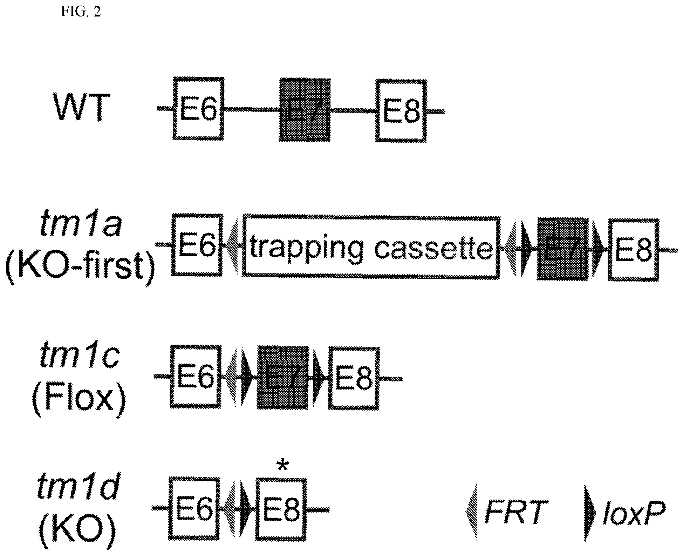

Mouse models for genetically knocking out STXBP1 were developed and are described in FIG. 2. The tmla allele has a trapping cassette that truncates transcription of the *Stxbp1* gene before exon 7. The trapping cassette can be removed with Flp mediated recombination to generate the tm1c allele, which has a floxed exon 7. Finally, exon 7 can be removed with a Cre recombinase to generate the tm1d allele, which has a non-viable *Stxbp1* gene.

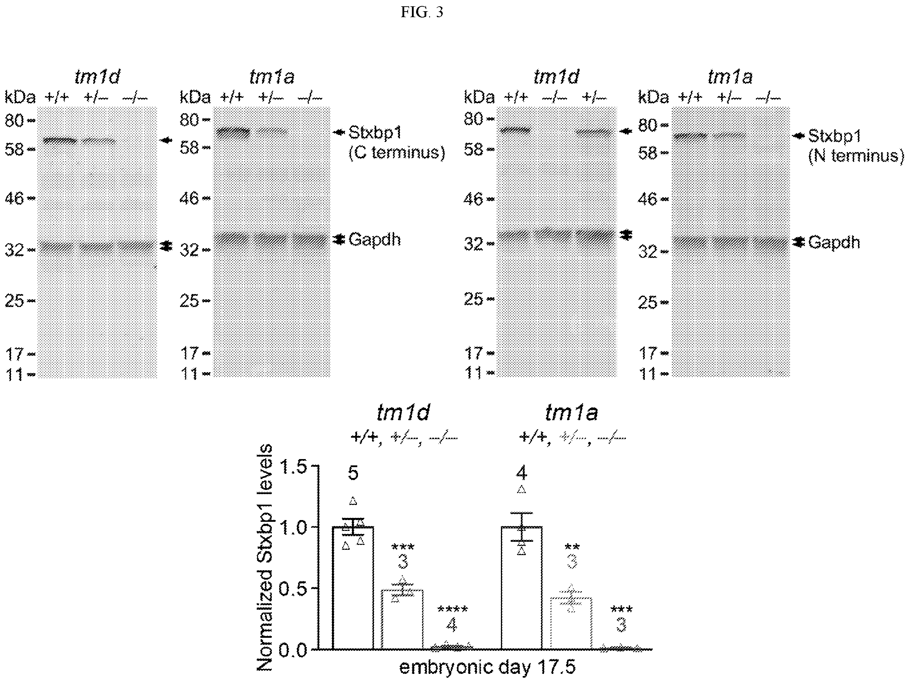

Mice with heterozygous tmla or tm1d alleles had approximately 50% STXBP1 protein expressed compared to wild-type mice (FIG. 3). Mice with homozygous tmla or tm1d had little to no detectable STXBP1 protein (FIG. 3).

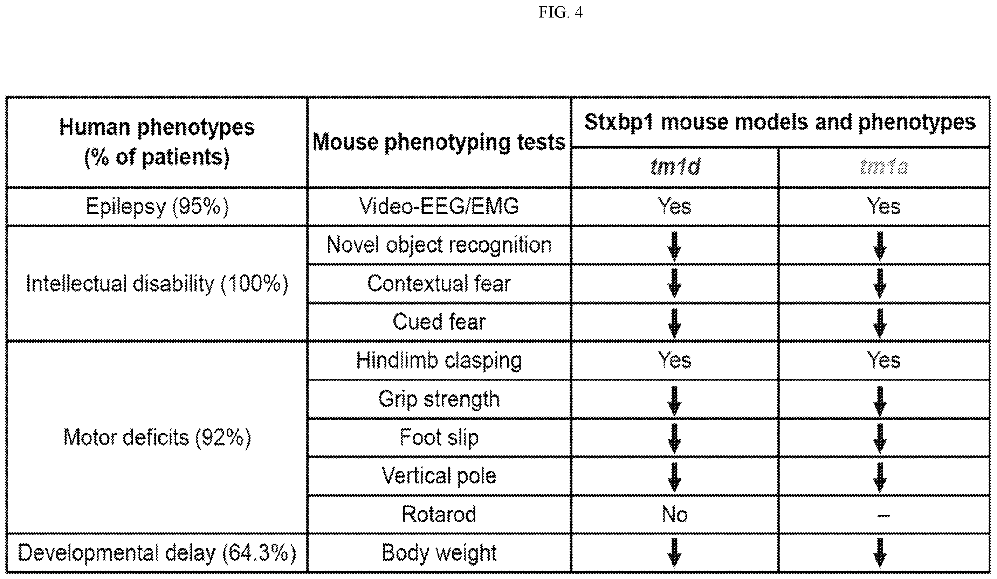

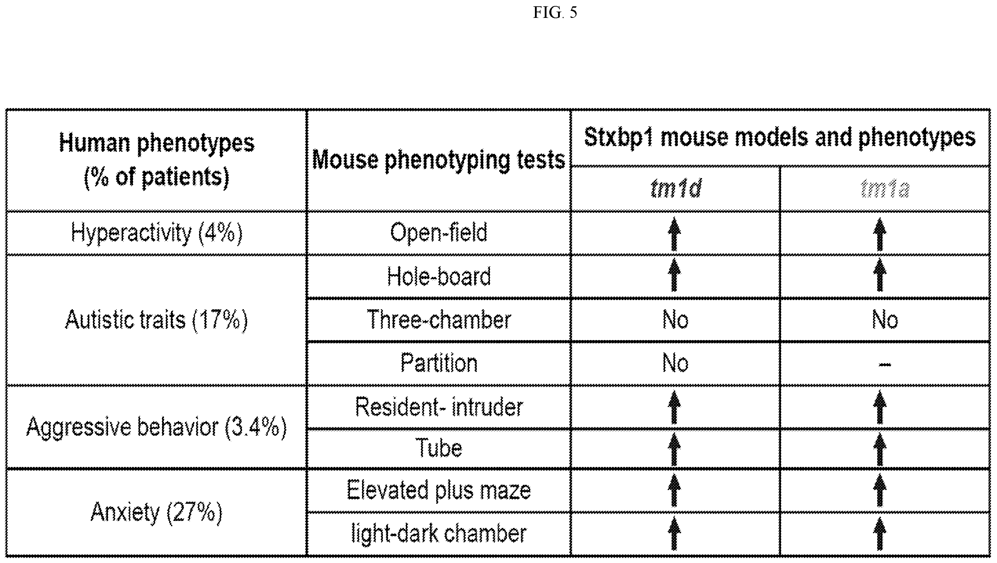

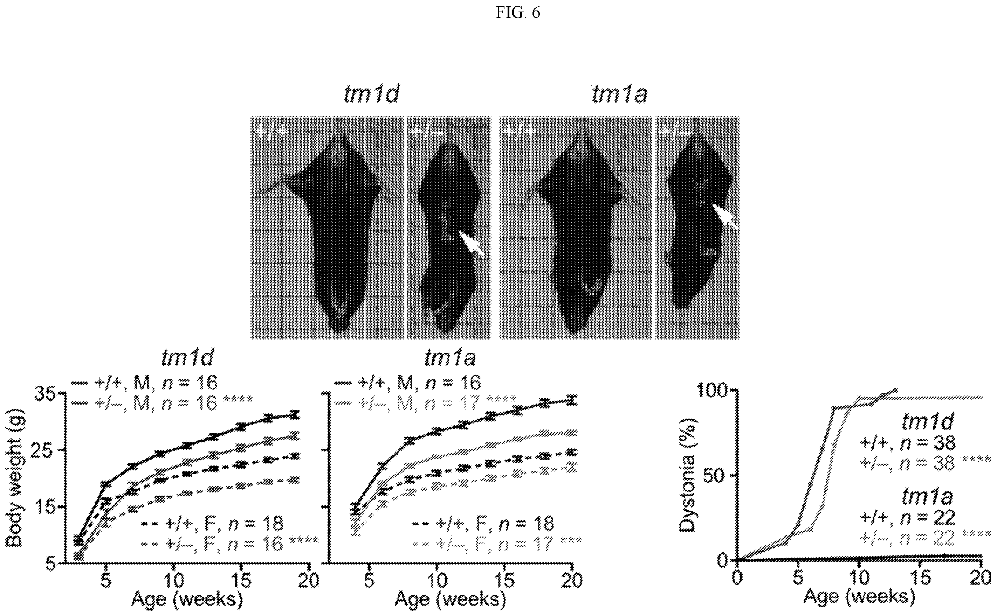

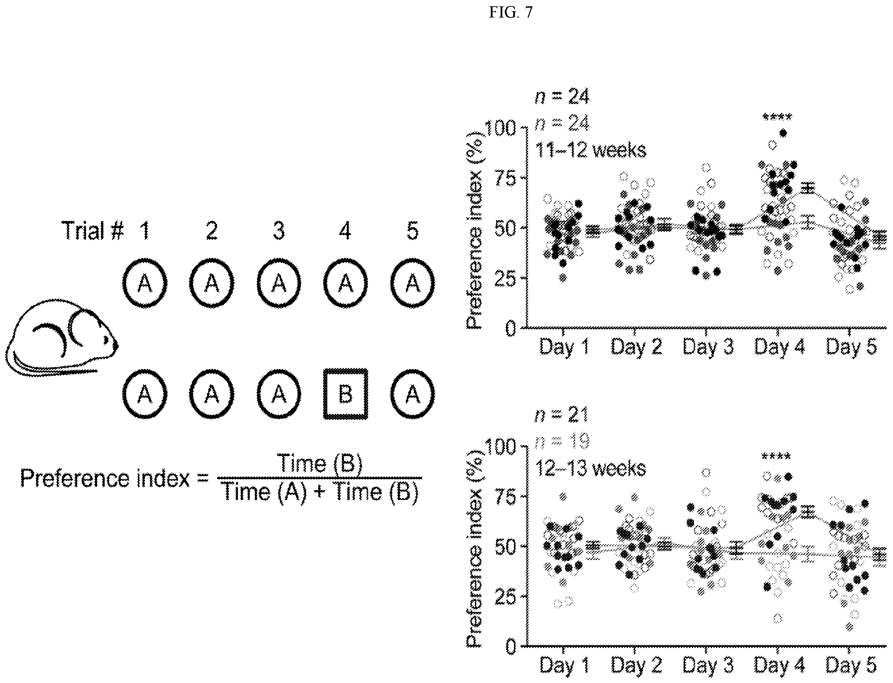

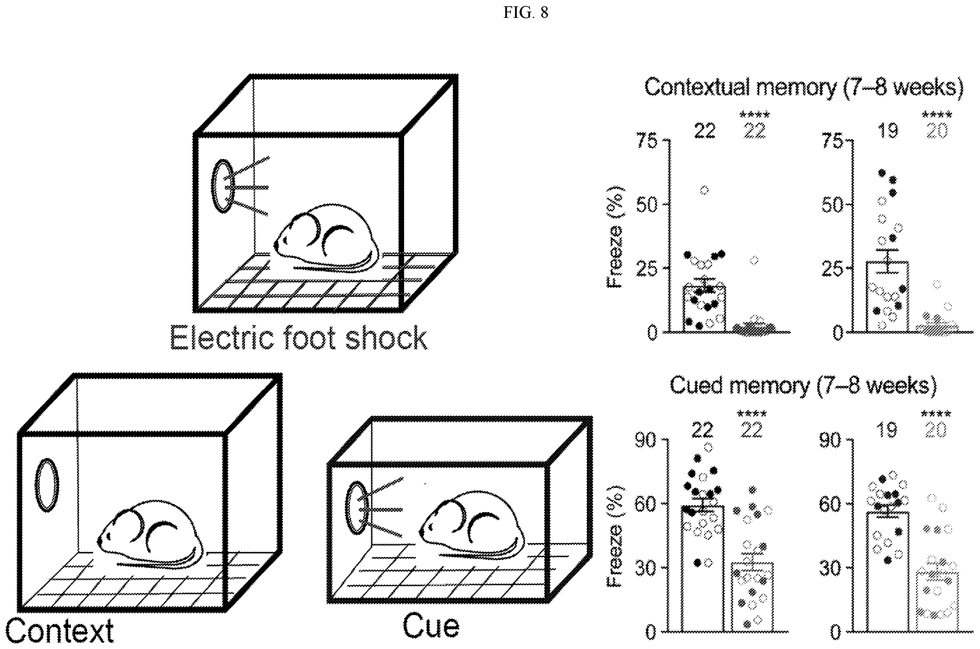

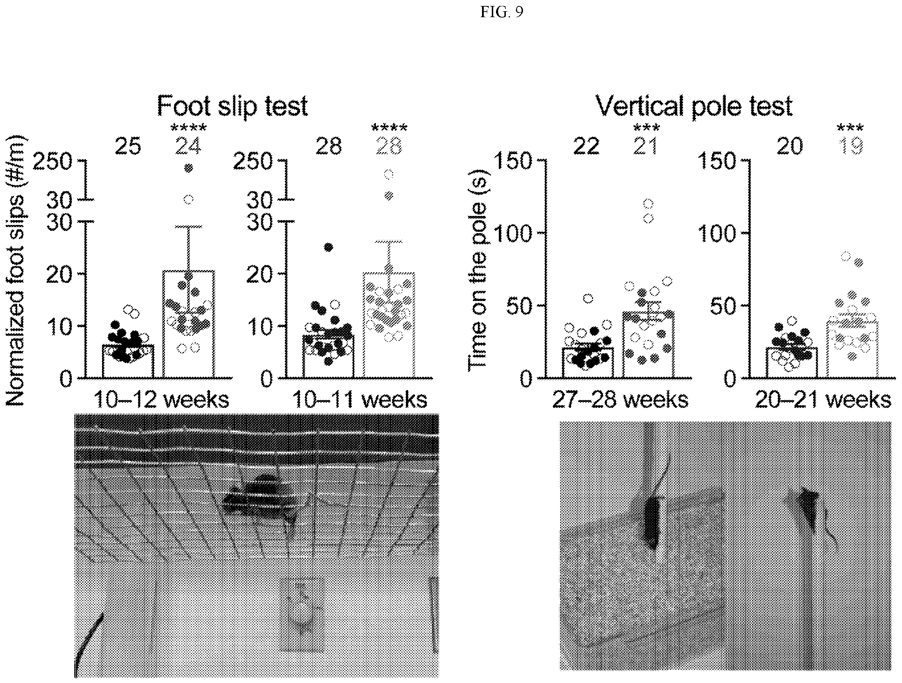

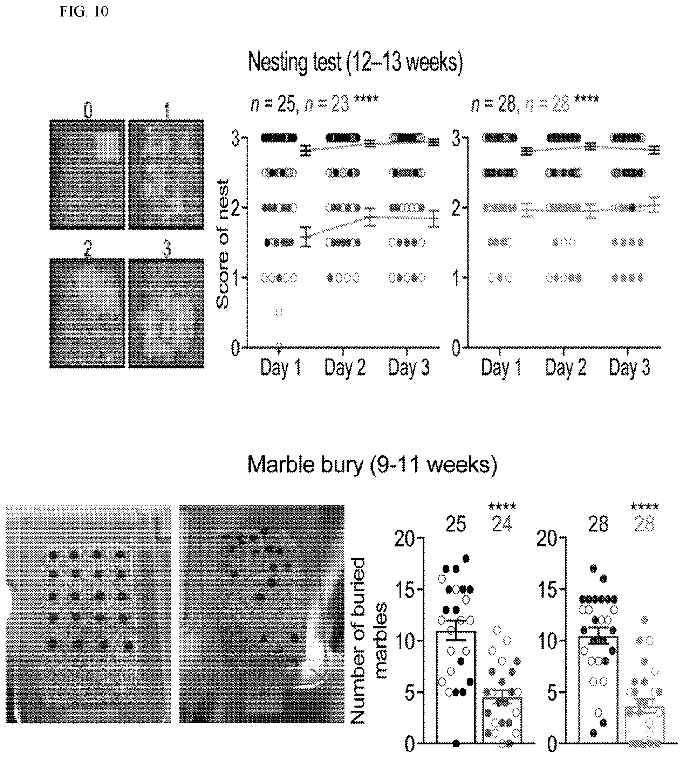

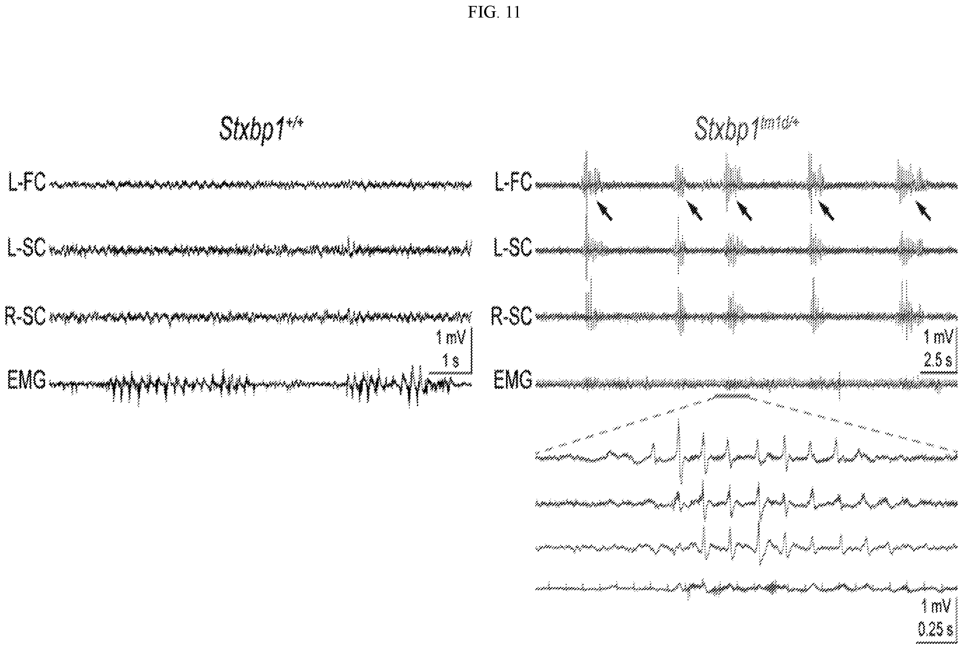

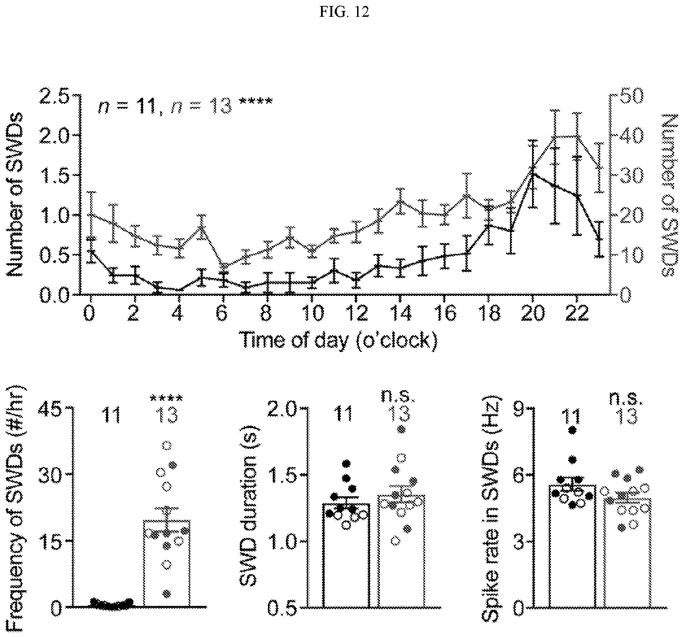

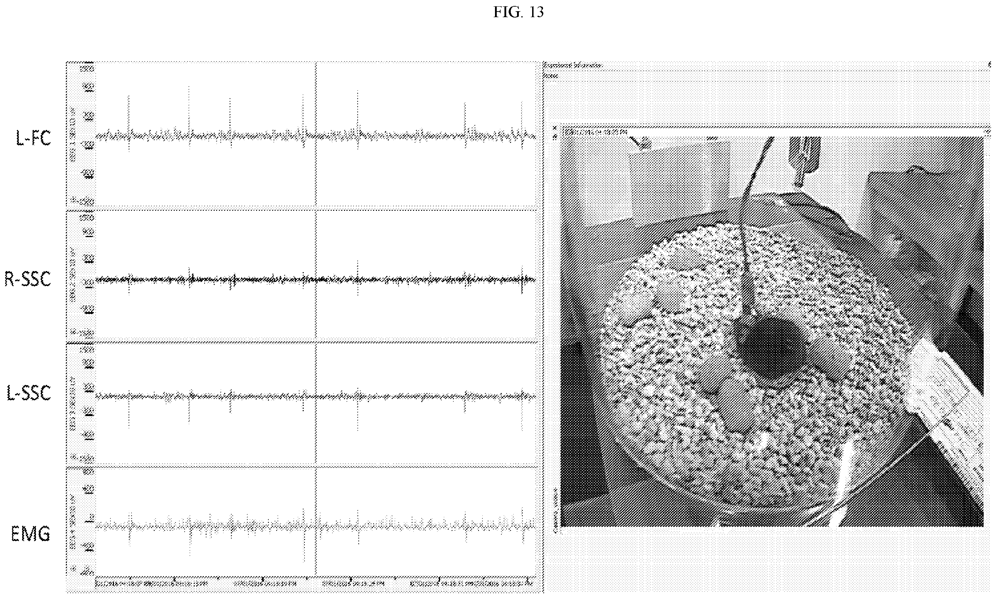

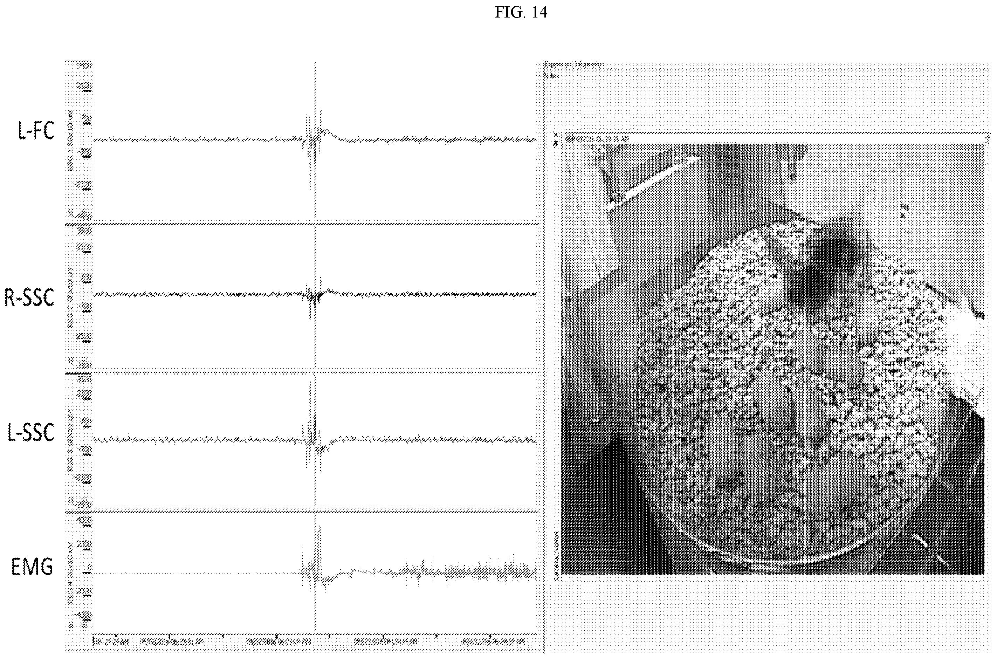

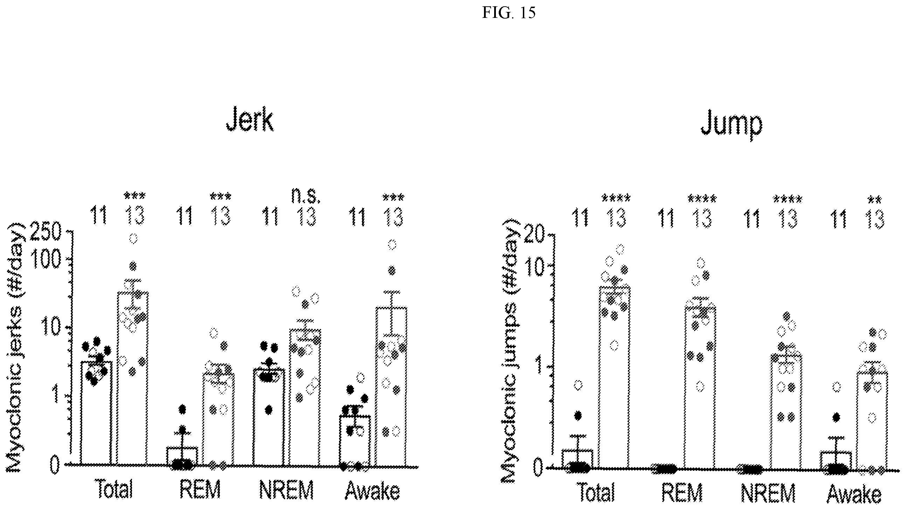

Mice with heterozygous tmla or tm1d alleles were observed to have epilepsy, intellectual disabilities, motor deficits, and developmental delays (FIG. 4). Mice with heterozygous tmla or tm1d alleles were also observed to have hyperactivity, autistic traits, aggressive behavior, and anxiety (FIG. 5). Mice with heterozygous tmla or tm1d alleles had reduced body weights compared to wild-type mice and displayed hindlimb clasping (FIG. 6). Mice with heterozygous tmla or tm1d alleles displayed cognitive defects in a novel object recognition test (FIG. 7). Mice with heterozygous tmla or tm1d alleles show contextual and cued memory defects in Pavlovian fear conditioning compared to wild-type mice (FIG. 8). Mice with heterozygous tmla or tm1d alleles displayed motor defects in a foot slip test and a vertical pole test (FIG. 9). Mice with heterozygous tmla or tm1d alleles displayed innate psychiatric defects in a nesting test and a marble bury test (FIG. 10). Heterozygous tm1d mice had more frequent SWDs compared to wild-type mice (FIG. 11 and FIG. 12). Heterozygous tm1d mice had more frequent myoclonic jerks and jumps compared to wild-type mice (FIGS. 13-15).

### EXAMPLE 2

### A VIRAL-GENETIC APPROACH TO RESTORE STXBP1 EXPRESSION IN ADULT MICE

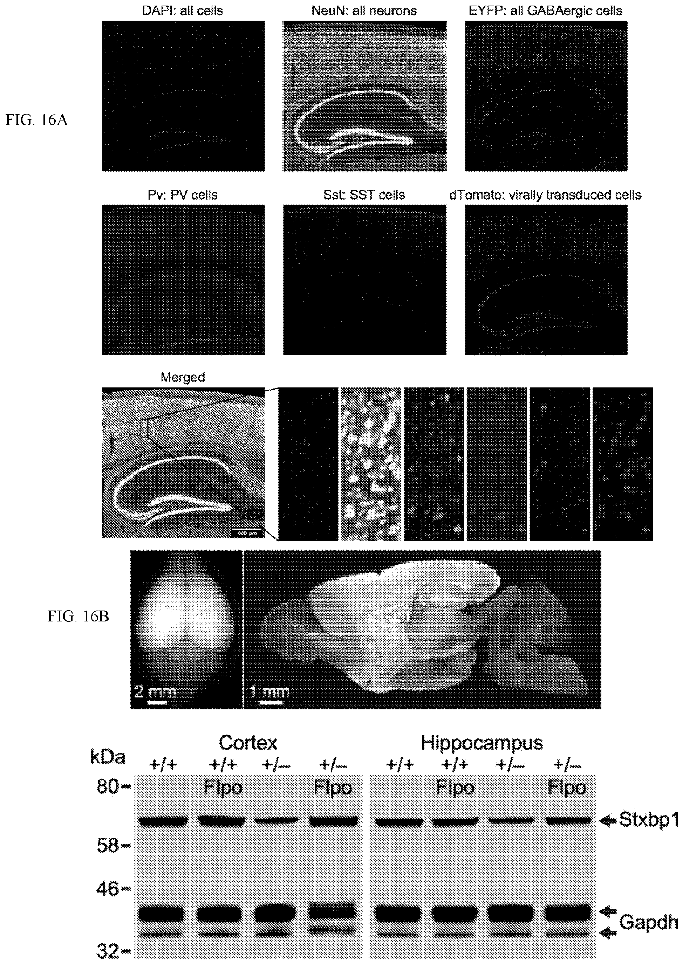

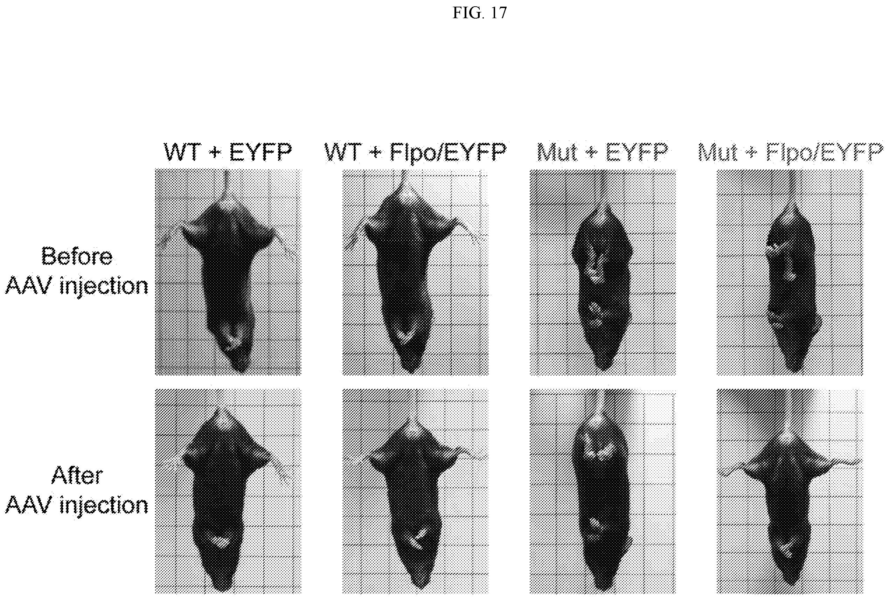

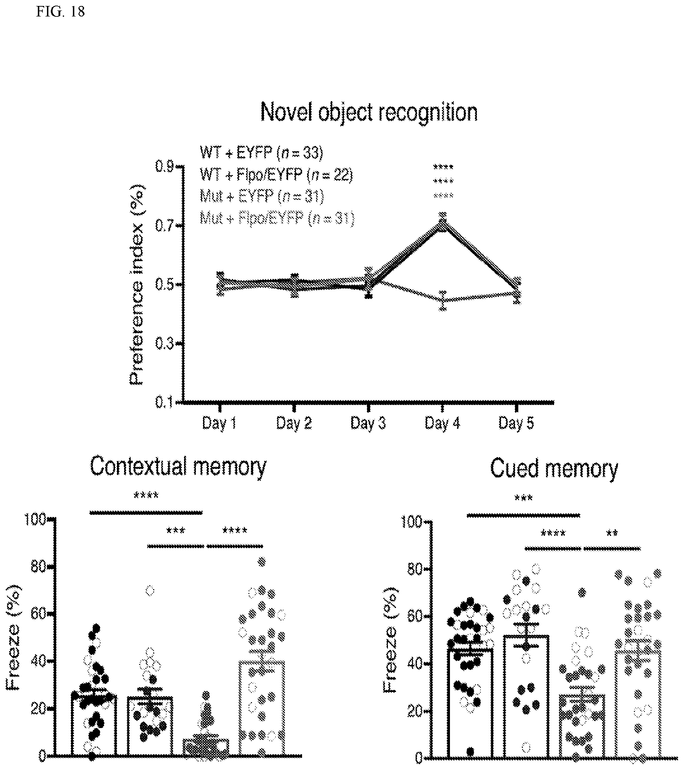

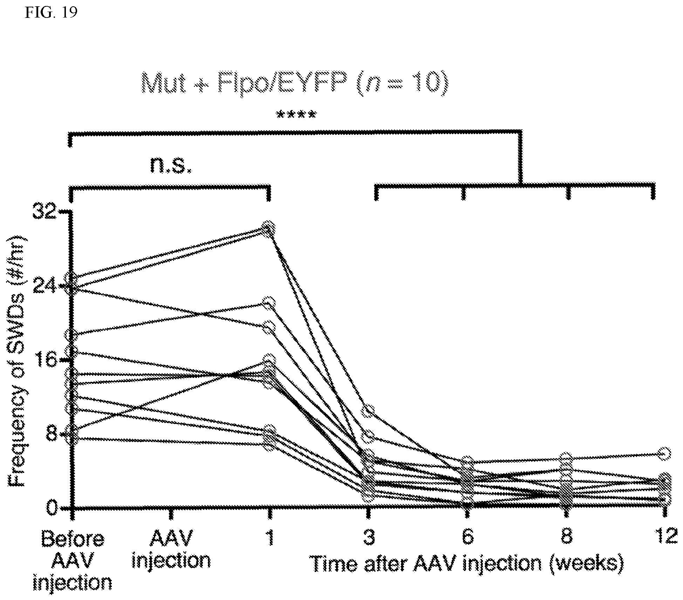

AAV-PHP.eB-NLS-dTomato was injected into 8-week-old *Vgat-ires-Cre;Rosa26-LSL-EYFP* mice to determine which cells are transduced by AAV-PHP.eB. Brain sections were stained with DAPI (labeling all cells), NeuN (labeling all neurons), Pv (labeling all PV-expressing neurons), and Sst (labeling all SST-expressing neurons). EYFP labels all GABAergic neurons and dTomato labels all virally transduced neurons, including glutamatergic neurons (*i.e.,* EYFP negative neurons), PV-expressing neurons (a subtype of GABAergic neurons), SST-expressing neurons (a subtype of GABAergic neurons), and other GABAergic neurons (FIG. 16A). Heterozygous tmla mice, relative to wild-type mice, showed a decrease in STXBP1 protein levels, which was restored with an AAV-PHP.eB that introduced an Flp recombinase (Flpo) to remove the trapping cassette in the tmla allele in 8-week-old mice (FIG. 16B). Flpo treatment in tmla mice reversed hindlimb clasping, rescued cognitive defects, and reduced SWDs compared to vehicle-treated tmla mice (FIGS. 17-19).

### EXAMPLE 3

### A GENE THERAPY APPROACH TO INCREASE STXBP1 LEVELS IN ADULT MICE

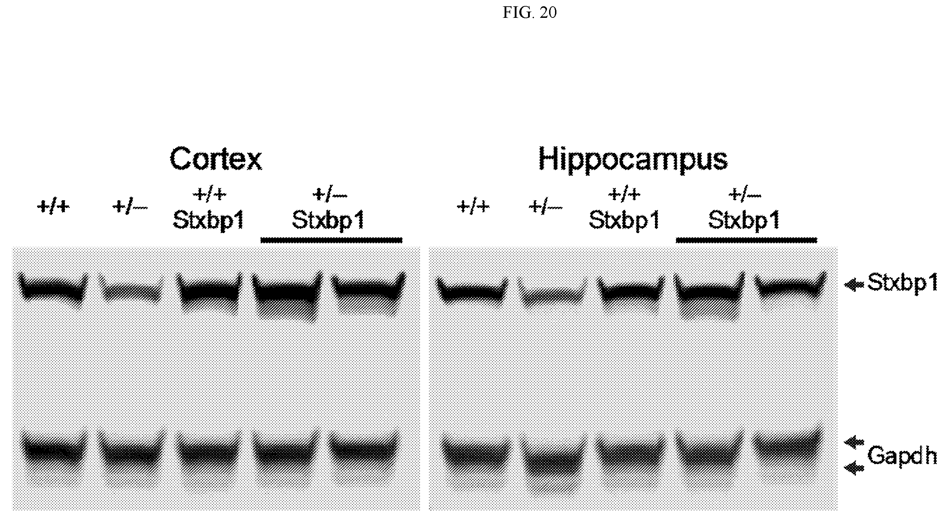

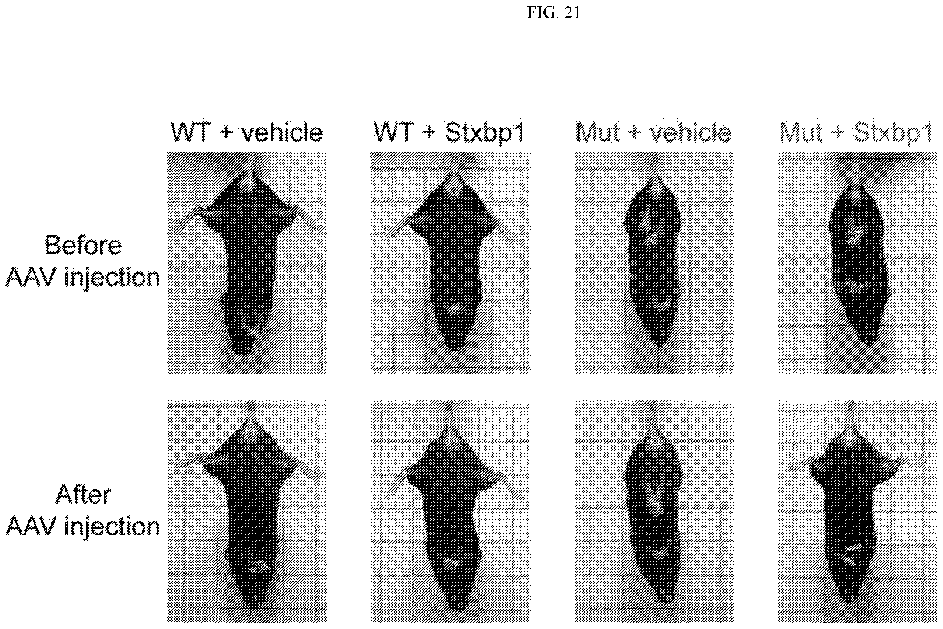

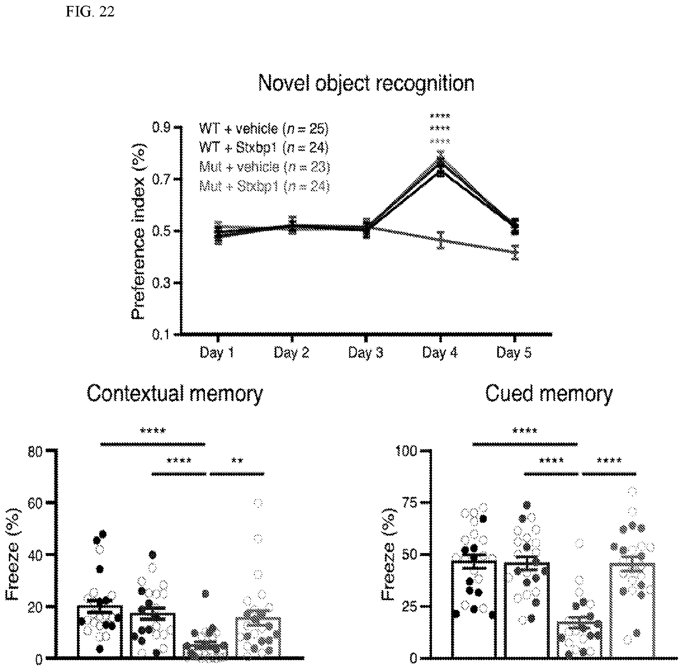

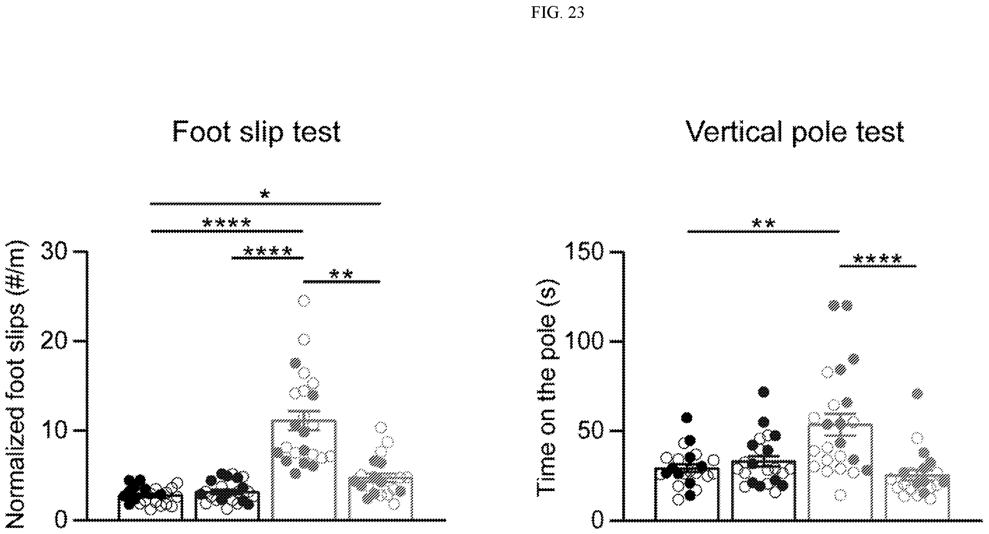

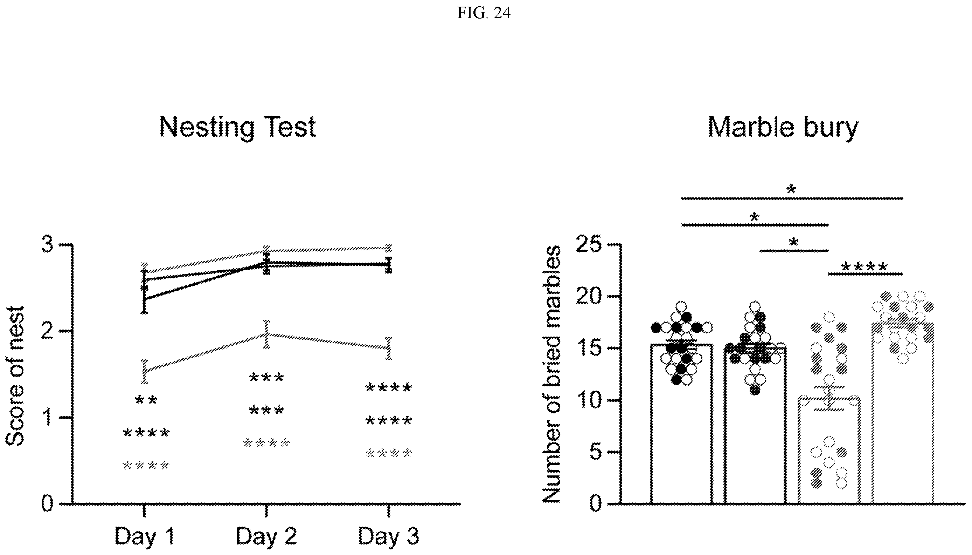

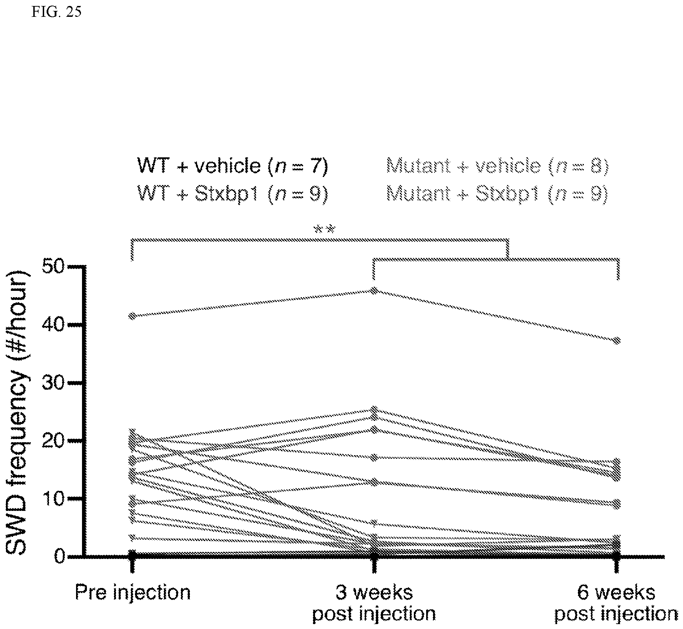

8-week-old heterozygous tm1d mice were injected with an AAV-PHP.eB comprising an *STXBP1* transgene (AAV-PHP.eB-STXBP1). The AAV restored STXBP1 protein level in tm1d mice to the level present in wild-type mice (FIG. 20). AAV-PHP.eB-STXBP1 treatment in tm1d mice reversed hindlimb clasping, rescued cognitive, motor, and innate psychiatric defects, and reduced SWDs compared to vehicle-treated tm1d mice (FIGS. 21-25).

### EXAMPLE 4

### CELL TYPE-SPECIFIC STXBP1 HETEROZYGOUS KNOCKOUT

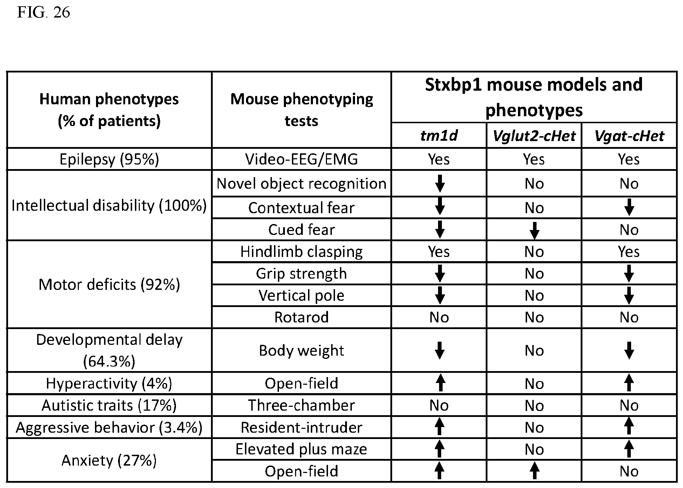

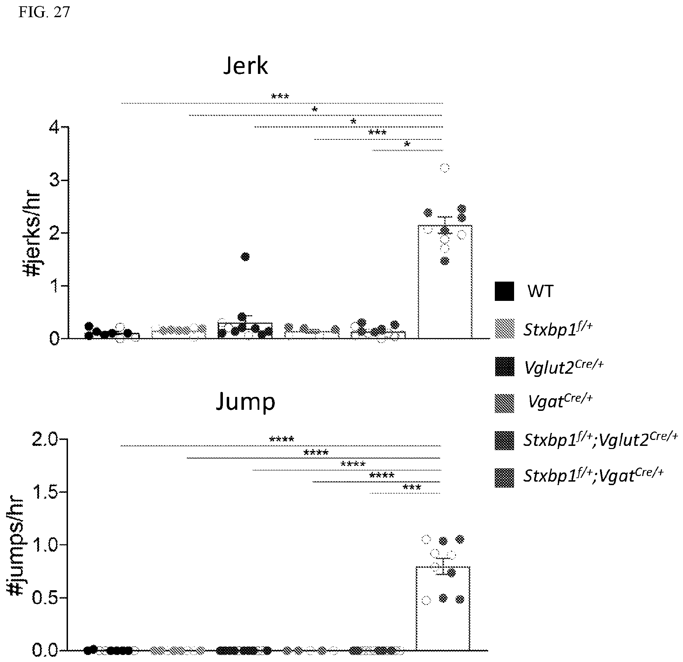

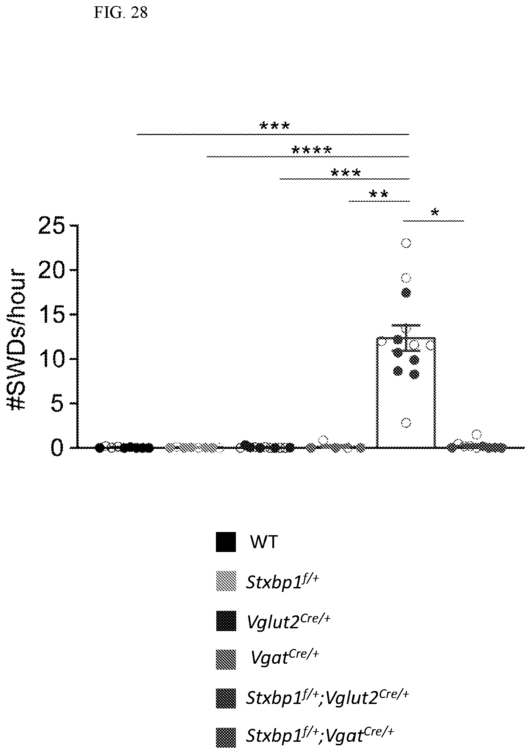

*Stxbp1-flox* mice were crossed with *Vglut2-ires-Cre* or *Vgat-ires-Cre* mice to generated mice with heterozygous knockouts of *Stxbp1* in glutamatergic or GABAergic neurons respectively. Heterozygous knockout in either glutamatergic (*Vglut2-cHet*) or GABAergic (*Vgat-cHet*) contributes to features of STXBP1 encephalopathy (FIG. 26). Frequent myoclonic seizures were observed in GABAergic but not glutamatergic-specific heterozygous *Stxbp1* knockout mice (FIG. 27), whereas frequent SWDs were observed in glutamatergic but not GABAergic-specific heterozygous *Stxbp1* knockout mice (FIG. 28).

Although the present disclosure and its advantages have been described in detail, it should be understood that various changes, substitutions and alterations can be made herein without departing from the spirit and scope of the design as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the present disclosure, processes, machines, manufacture, compositions of matter, means, methods, or steps, presently existing or later to be developed that perform substantially the same function or achieve substantially the same result as the corresponding embodiments described herein may be utilized according to the present disclosure. Accordingly, the appended claims are intended to include within their scope such processes, machines, manufacture, compositions of matter, means, methods, or steps.

### FURTHER ASPECTS OF THE DISCLOSURE

1. An adeno-associated virus (AAV) particle comprising a nucleic acid comprising a sequence encoding an STXBP1 gene product.
2. The AAV particle of aspect 1, wherein the AAV particle is an AAV-PHP.eB particle, AAV-PHP.B particle, AAV-PHP.S particle, AAV-PHP.B4 particle, AAV-PHP.B5 particle, AAV-PHP.N particle, AAV-CAP-B1 particle, AAV-CAP-B10 particle, AAV-CAP-B22 particle, AAV type 1 particle, AAV type 5 particle, AAV type 8 particle, or AAV type 9 particle.
3. The AAV particle of aspect 2, wherein the AAV particle is an AAV-PHP.eB particle, AAV-CAP-B10 particle, AAV-CAP-B22 particle, or AAV type 9 particle.
4. The AAV particle of any one of aspects 1-3, wherein the STXBP1 gene product comprises an amino acid sequence having at least 90% sequence identity with SEQ ID NO:1.
5. The AAV particle of any one of aspects 1-3, wherein the STXBP1 gene product comprises the amino acid sequence of SEQ ID NO:1.
6. The AAV particle of any one of aspects 1-3, wherein the STXBP1 gene product comprises an amino acid sequence having at least 90% sequence identity with SEQ ID NO:2.
7. The AAV particle of any one of aspects 1-3, wherein the STXBP1 gene product comprises the amino acid sequence of SEQ ID NO:2.
8. The AAV particle of any one of aspects 1-3, wherein the STXBP1 gene product comprises an amino acid sequence having at least 90% sequence identity with SEQ ID NO:3.
9. The AAV particle of any one of aspects 1-3, wherein the STXBP1 gene product comprises the amino acid sequence of SEQ ID NO:3.
10. The AAV particle of any one of aspects 1-3, wherein the STXBP1 gene product comprises an amino acid sequence having at least 90% sequence identity with SEQ ID NO:4.
11. The AAV particle of any one of aspects 1-3, wherein the STXBP1 gene product comprises the amino acid sequence of SEQ ID NO:4.
12. The AAV particle of any one of aspects 1-11, wherein the sequence encoding the STXBP1 gene product lacks an intron.
13. The AAV particle of any one of aspects 1-12, wherein the sequence encoding the STXBP1 gene product is operably linked to one or more regulatory sequences.
14. The AAV particle of aspect 13, wherein the one or more regulatory sequences induce expression of the STXBP1 gene product in cells of the central and/or peripheral nervous system.
15. The AAV particle of aspect 13 or 14, wherein the one or more regulatory sequences comprise a promoter selected from the group consisting of CAG, CBA, EF1a, UbiC, CMV, hSyn1, MeCP2, NSE, BM88, Vglut1, Vglut2, CaMKII, Vgat, DLX5/6, mDlx, NR2E1, and a combination thereof.
16. The AAV particle of aspect 14 or 15, wherein the cells of the central and/or peripheral nervous system comprise glutamatergic neurons, GABAergic neurons, glycinergic neurons, cholinergic neurons, dopaminergic neurons, serotonergic neurons, adrenergic neurons, and/or noradrenergic neurons.
17. A method of treating an individual with an encephalopathy comprising administering a therapeutically effective amount of the AAV particle of any one of aspects 1-16 to the individual.
18. The method of aspect 17, wherein the encephalopathy is STXBP1 encephalopathy or early infantile epileptic encephalopathy type 4.
19. The method of aspect 18, wherein the encephalopathy is STXBP1 encephalopathy.
20. The method of any of aspects 17-19, wherein the individual has Ohtahara syndrome, West syndrome, Lennox-Gastaut syndrome, Dravet syndrome, or Rett syndrome with mutated *STXBP1.*
21. The method of any one of aspects 17-20, wherein the individual has one or more mutations in a *STABP1* gene.
22. The method of any one of aspects 17-21, wherein individual has one or more missense mutations, nonsense mutations, deletions, inversions, insertions, duplications, frameshift mutations, repeat expansions, haploinsufficiencies, or a combination thereof in the *STXBP1* gene.
23. The method of any one of aspects 17-22, wherein the individual is an infant, child, or adolescent.
24. The method of any one of aspects 17-22, wherein the individual is an adult.
25. The method of any one of aspects 17-24, wherein the AAV particle is administered by injection into brain tissue.
26. The method of any one of aspects 17-24, wherein the AAV particle is administered by injection outside of the brain.
27. The method of any of aspects 17-26, further comprising, prior to administering the AAV particle to the individual, detecting a mutation in *STXBP1* in the individual.
28. An AAV particle comprising a nucleic acid encoding an STXBP1 gene product having at least 90% sequence identity with SEQ ID NO:2.
29. The AAV particle of aspect 28, wherein the STXBP1 gene product has at least 95% sequence identity with SEQ ID NO:2.
30. The AAV particle of aspect 28, wherein the STXBP1 gene product comprises SEQ ID NO:2.
31. A method of treating an individual with an encephalopathy comprising administering a therapeutically effective amount of the AAV particle of any of aspects 28-30.
32. A method of treating an individual with an STXBP1 encephalopathy comprising administering a therapeutically effective amount of an AAV particle comprising a nucleic acid encoding an STXBP1 gene product having at least 95% sequence identity with SEQ ID NO:2.
33. The method of aspect 32, wherein the STXBP1 gene product comprises SEQ ID NO:2.
34. The AAV particle of aspect 1, wherein the AAV particle is a particle described in WO2020206189, WO2020028751, WO2020168145, WO2020077165, WO2021025995, WO2017197355, WO2018022905, WO201700671, WO2017218842, WO2016154344, WO2017192750; WO2016081811, WO2019222329 or WO2019028306.
35. The AAV particle of aspect 1, wherein the particle comprises one or more of SEQ ID NOs: 15-16 of PCT patent application publication WO2020206189; SEQ ID NOs: 4-14326 and 42973-42999 of PCT patent application publication WO2020028751; SEQ ID NO: 1829 of PCT patent application publication WO2020077165; SEQ ID NO: 43073-43341 of PCT patent application publication WO2021025995; SEQ ID NOs: 14-41 of PCT patent application publication WO2017197355; SEQ ID NOs: 47-142 of PCT patent application publication WO2018022905; SEQ ID NO: 37 of PCT patent application publication WO201700671; SEQ ID NOs: 3-78 of PCT patent application publication WO2017218842; SEQ ID NOs: 8-14 of PCT patent application publication WO2017192750; SEQ ID NOs: 1-43 of PCT patent application publication WO2016081811; SEQ ID NOs: 1824-1825 of PCT patent application publication WO2019222329; and SEQ ID NOs: 1809-1810 of PCT patent application publication WO2019028306.

## Claims

1. An adeno-associated virus (AAV) particle comprising a nucleic acid encoding an STXBP1 gene product having at least 90% sequence identity with SEQ ID NO:2.

2. The AAV particle of claim 1, wherein the STXBP1 gene product:
(a) has at least 95% sequence identity with SEQ ID NO:2; or
(b) comprises SEQ ID NO:2.

3. The AAV particle of claim 1 or 2, for use in a method of treating an individual with an encephalopathy comprising administering a therapeutically effective amount of the AAV particle.

4. An AAV particle comprising a nucleic acid encoding an STXBP1 gene product having at least 95% sequence identity with SEQ ID NO:2 for use in a method of treating an individual with an STXBP1 encephalopathy comprising administering a therapeutically effective amount of said AAV particle.

5. The AAV particle for use of claim 4, wherein the STXBP1 gene product comprises SEQ ID NO:2.

6. An adeno-associated virus (AAV) particle comprising a nucleic acid comprising a sequence encoding an STXBP1 gene product.

7. The AAV particle of claim 6, wherein the AAV particle is an AAV-PHP.eB particle, AAV-PHP.B particle, AAV-PHP.S particle, AAV-PHP.B4 particle, AAV-PHP.B5 particle, AAV-PHP.N particle, AAV-CAP-B1 particle, AAV-CAP-B10 particle, AAV-CAP-B22 particle, AAV type 1 particle, AAV type 5 particle, AAV type 8 particle, or AAV type 9 particle.

8. The AAV particle of claim 7, wherein the AAV particle is an AAV-PHP.eB particle, AAV-CAP-B10 particle, AAV-CAP-B22 particle, or AAV type 9 particle.

9. The AAV particle of any one of claims 6-8, wherein the STXBP1 gene product comprises:
(a) an amino acid sequence having at least 90% sequence identity with SEQ ID NO:2; or
(b) the amino acid sequence of SEQ ID NO:2; or
(c) an amino acid sequence having at least 90% sequence identity with SEQ ID NO:1; or
(d) the amino acid sequence of SEQ ID NO:1; or
(e) an amino acid sequence having at least 90% sequence identity with SEQ ID NO:3; or
(f) the amino acid sequence of SEQ ID NO:3; or
(g) an amino acid sequence having at least 90% sequence identity with SEQ ID NO:4; or
(h) the amino acid sequence of SEQ ID NO:4.

10. The AAV particle of any one of claims 6-9, wherein the sequence encoding the STXBP1 gene product lacks an intron.

11. The AAV particle of any one of claims 6-10, wherein the sequence encoding the STXBP1 gene product is operably linked to one or more regulatory sequences, optionally wherein:
(i) the one or more regulatory sequences induce expression of the STXBP1 gene product in cells of the central and/or peripheral nervous system; and/or
(ii) the one or more regulatory sequences comprise a promoter selected from the group consisting of CAG, CBA, EF1a, UbiC, CMV, hSyn1, MeCP2, NSE, BM88, Vglut1, Vglut2, CaMKII, Vgat, DLX5/6, mDlx, NR2E1, and a combination thereof.

12. The AAV particle of claim 11(i) or 11(ii), wherein the cells of the central and/or peripheral nervous system comprise glutamatergic neurons, GABAergic neurons, glycinergic neurons, cholinergic neurons, dopaminergic neurons, serotonergic neurons, adrenergic neurons, and/or noradrenergic neurons.

13. The AAV particle of any one of claims 6-12, for use in a method of treating an individual with an encephalopathy comprising administering a therapeutically effective amount of the AAV particle to the individual.

14. The AAV particle for use of claim 13, wherein:
(a) the encephalopathy is STXBP1 encephalopathy or early infantile epileptic encephalopathy type 4; and/or
(b) the individual has Ohtahara syndrome, West syndrome, Lennox-Gastaut syndrome, Dravet syndrome, or Rett syndrome with mutated *STXBP1;* and/or
(c) the individual has one or more mutations in a *STIBP1* gene; and/or
(d) individual has one or more missense mutations, nonsense mutations, deletions, inversions, insertions, duplications, frameshift mutations, repeat expansions, haploinsufficiencies, or a combination thereof in the *STIBP1* gene; and/or
(e) the individual is: (i) an infant, child, or adolescent; or (ii) an adult; and/or
(f) the AAV particle is administered: (i) by injection into brain tissue; or (ii) by injection outside of the brain; and/or
(g) the method further comprises, prior to administering the AAV particle to the individual, detecting a mutation in *STXBP1* in the individual.

15. The AAV particle of claim 6, wherein:
(a) the AAV particle is a particle described in WO2020206189, WO2020028751, WO2020168145, WO2020077165, WO2021025995, WO2017197355, WO2018022905, WO201700671, WO2017218842, WO2016154344, WO2017192750; WO2016081811, WO2019222329 or WO2019028306; or
(b) the particle comprises one or more of SEQ ID NOs: 15-16 of PCT patent application publication WO2020206189; SEQ ID NOs: 4-14326 and 42973-42999 of PCT patent application publication WO2020028751; SEQ ID NO: 1829 of PCT patent application publication WO2020077165; SEQ ID NO: 43073-43341 of PCT patent application publication WO2021025995; SEQ ID NOs: 14-41 of PCT patent application publication WO2017197355; SEQ ID NOs: 47-142 of PCT patent application publication WO2018022905; SEQ ID NO: 37 of PCT patent application publication WO201700671; SEQ ID NOs: 3-78 of PCT patent application publication WO2017218842; SEQ ID NOs: 8-14 of PCT patent application publication WO2017192750; SEQ ID NOs: 1-43 of PCT patent application publication WO2016081811; SEQ ID NOs: 1824-1825 of PCT patent application publication WO2019222329; and SEQ ID NOs: 1809-1810 of PCT patent application publication WO2019028306.
